# EUROPEAN PATENT APPLICATION

(11) **EP 2 722 031 A1**
(43) Date of publication of application: **23.04.2014**
(21) Application number: 13189023.8
(22) Date of filing: 17.10.2013
(51) Int. Cl.: A61J 7/00, B65D 75/42, G07F 11/68, A61J 7/04, B65H 35/00

(54) **Medicine-taking management apparatus**

(30) Priority: 18.10.2012 KR 20120115838; 18.10.2012 KR 20120115839; 18.10.2012 KR 20120115840; 02.10.2013 KR 20130117794
(71) Applicant: JVM Co., Ltd., Daegu 704-947 (KR)
(72) Inventor: Kim, Jun Ho, 706-822 Daegu (KR)
(74) Representative: Dantz, Jan Henning

(57) **Abstract**

Provided is a medicine-taking management apparatus. The medicine-taking management apparatus includes a preservation unit for preserving a chartula package, a transfer unit including at least one roll for transferring the preserved chartula package, a cutting unit for cutting a chartula of the transferred chartula package, a dispensing hole through which the cut chartula is dispensed from the chartula package to the outside, a storage unit providing a space in which the cut chartula is stored therein, and a path selection unit for controlling a path so that the cut chartula travels to one of the dispensing hole and the storage unit. Even though the medicine is not taken at a fixed medicine-taking time when the medicine-taking time is fixed, the follow-up medicine-taking management may be enabled without separately managing the corresponding chartula by the user.

## Description

### CROSS-REFERENCE TO RELATED PATENT APPLICATION

This application claims the benefit of Korean Patent Application Nos. 10-2012-0115838, 10-2012-0115839, 10-2012-0115840 filed on October 18, 2012 and 10-2013-0117794 filed on October 2, 2013 in the Korean Intellectual Property Office, the disclosure of which is incorporated herein in its entirety by reference.

### BACKGROUND OF THE INVENTION

### 1. Field of the Invention

The present invention relates to a medicine-taking management apparatus, and more particularly, to an apparatus for managing the dispensing of a chartula package according to prescription and medicine-taking information.

### 2. Description of the Related Art

In general, for patients who should take medicines for a long time, it is important to timely take the required dose of medical preparations according to prescription, above all things. Patients often forget the administration time or administration dose of medicine, or may often take an overdose of medicine, which had to be administered already, at one time after the lapse of the administration time, without any consultation with a doctor or pharmacist. Also, patients such as the chronically ill often intentionally avoid the administration of medicine because of their fearful mind of side effects caused by a long-term administration of medicines. Such an irregular administration of medicines may cause the compliance of medicine administration for patients to be degraded, or the duration required for treatment of diseases to be prolonged, and may also lead to a decrease in therapeutic efficacy.

A medical team is unable to check one by one administration states of patients who receive medical attention at home instead of a hospital, and furthermore it is difficult to systematically control therapeutic states of patients.

As an exemplary approach to overcome such limitations, Korean Utility Model No. 20-0201878, entitled "Apparatus of Notifying Administration Time of Medicine" (hereinafter, referred to as 'related art') was proposed. The related art is advantageous in that melody sound or flickering warning lamp notifies patients of the administration time of medicines prescribed. However, the related art is still disadvantageous because protectors or the like are unable to check how to handle medicines which had to be administered already or whether medicines were administered or not.

### SUMMARY OF THE INVENTION

The present invention provides an apparatus for allow a user to take a medicine at an adequate medicine-taking time.

The present invention also provides a medicine-taking management apparatus for automatically managing a chartula of a medicine that is not taken at a medicine-taking time without separately managing the chartula by a user.

The present invention also provides a medicine-taking management apparatus providing additional information that is of help to determine medicine-taking history information.

The present invention also provides a medicine-taking management apparatus having a structure that is capable of be easily transferred to dispense a chartula package.

The present invention also provides a medicine-taking management apparatus having a structure that is capable of easily supplementing or replacing a chartula package.

The technical objects of the present invention are not limited to those described above, and it will be apparent to those of ordinary skill in the art from the following description that the present invention includes other technical objects not specifically mentioned herein.

According to an aspect of the present invention, there is provided a medicine-taking management apparatus including: a preservation unit for preserving a chartula package; a transfer unit including at least one roll for transferring the preserved chartula package; a cutting unit for cutting a chartula of the transferred chartula package; a dispensing hole through which the cut chartula is dispensed from the chartula package to the outside; a storage unit providing a space in which the cut chartula is stored therein; and a path selection unit for controlling a path so that the cut chartula travels to one of the dispensing hole and the storage unit.

The medicine-taking management apparatus may further include a housing including a detachment part to which the preservation unit is detached.

The preservation unit may be provided in plurality with various sizes and shapes according to the number of days of prescription, and the plurality of preservation units may be selectively mounted on the housing.

The path selection unit may include: a support plate providing a path so that the cut chartula is transferred up to the dispensing hole; a rotation shaft disposed parallel to a transfer direction of the cut chartula; and a connection arm connecting the rotation shaft to the support plate, the connection arm being rotated so that the support plate is inclined at a predetermined angle or more.

The medicine-taking management apparatus may further include a transfer path support plate providing a path so that the cut chartula is transferred up to the dispensing hole. And the path selection unit may include: an extension part disposed between the transfer path support plate and the dispensing hole so that the transfer path extends; and a path selection driving part moving the extension part between the transfer path support plate and the dispensing hole to form a first transfer path of the cut chartula, which are defined by the transfer path support plate, the extension part, and the dispensing hole and separating the extension part from a space between the transfer path support plate and the dispensing hole to form a second transfer path for allowing the cut chartula to drop into the space between the transfer path support plate and the dispensing hole.

The medicine-taking management apparatus may further include a support part having an elevation guide groove having a groove shape with a predetermined length in a vertical direction, wherein the extension part may include a body part continuously forming the transfer path together with the support plate, a side part extending from each of both side surfaces of the body part, a guide protrusion protruding inward from the side part and guided in a state where the guide protrusion is inserted into the elevation guide groove, and a protrusion to be pressed, which protrudes outward from the side part, the path selection driving part may include a pair of rotation arms that are rotated about a rotation shaft disposed perpendicular to the transfer path and the elevation direction of the extension part, and each of the rotation arms may have a cutoff portion, of which a portion is cut to accommodate the protrusion to be pressed, on an outer end thereof.

The transfer unit may include: a discharge roll being rolled to discharge the cut chartula; and an unwinding roll for transferring the chartula package toward the dispensing hole, and at least one outer surface of the discharge roll and the unwinding roll may have an outer surface formed of a porous elastic material.

At least one the discharge roll and the unwinding roll may have a concave portion and a convex portion which are alternately disposed along an outer surface thereof.

The storage unit may include an inclined guide part so that the cut chartula dropping from the path selection unit is seated.

The medicine-taking management apparatus may further include a scan part for scanning a cutting position of the chartula package transferred by the transfer unit and a two-dimensional graphic code printed on the chartula package.

The medicine-taking management apparatus may further includes a control part for recognizing at least one medicine-taking information of patient identification data, a medicine-taking time, and chartula package information from the two-dimensional graphic code scanned by the scan part.

The control part may control the transfer unit, the cutting unit, and the path selection unit to store a corresponding chartula into the storage unit when a specific chartula corresponding to a specific medicine-taking time is not dispensed through the dispensing hole for the specific medicine-taking time.

The medicine-taking management apparatus may further include an imaging part for photographing an external image and communication partwherein the control part may include the medicine-taking information including the medicine-taking time and the chartula package information that are recognized from the two-dimensional graphic code scanned by the scan part to an actually dispensed time of the chartula through the dispensing hole and information of the actually dispensed chartula, and a communication part may transmit the result obtained by the comparing the medicine-taking information to the medicine-taking history information through the control part to at least one of a guardian terminal and a management server.

The transfer unit may be disposed so that the transfer path is inclined downward toward the dispensing hole.

The storage unit may be provided in a drawer type that is withdrawable to the outside.

### BRIEF DESCRIPTION OF THE DRAWINGS

The above and other features and advantages of the present invention will become more apparent by describing in detail exemplary embodiments thereof with reference to the attached drawings in which:
FIG. 1 is a block diagram illustrating the whole structure of a medicine-taking management system according to an embodiment of the present invention;
FIG. 2 is a perspective view of a medicine-taking management apparatus according to an embodiment;
FIG. 3 is a perspective view of a state in which an upper case of the medicine-taking management apparatus is separated according to an embodiment;
FIG. 4 is an exploded perspective view of the medicine-taking management apparatus according to an embodiment;
FIG. 5 is an exploded perspective view illustrating main components of the medicine-taking management apparatus according to an embodiment;
FIG. 6 is a cross-sectional view of the medicine-taking management apparatus according to an embodiment;
FIG. 7 is a cross-sectional view illustrating an initial process for mounting a chartula package on the medicine-taking management apparatus according to an embodiment;
FIG. 8 is a schematic view of the chartula package according to an embodiment;
FIG. 9 is a cross-sectional view illustrating a state in which the chartula package is transferred within the medicine-taking management apparatus according to an embodiment;
FIG. 10 is a cross-sectional view illustrating a state in which a portion of the chartula package is cut within the medicine-taking management apparatus according to an embodiment;
FIG. 11 is a partially enlarged cross-sectional view illustrating a state in which the portion of the chartula package of FIG. 10 is cut;
FIG. 12 is a cross-sectional view illustrating a state in which a chartula is dispensed through a first path of the medicine-taking management apparatus according to an embodiment;
FIG. 13 is a partial perspective view illustrating an operation process for selecting a second path within the medicine-taking management apparatus according to an embodiment;
FIG. 14 is a partial front view illustrating an operation process for selecting the second path within the medicine-taking management apparatus according to an embodiment;
FIG. 15 is a cross-sectional view illustrating a state in which a chartula is stored through the second path within the medicine-taking management apparatus according to an embodiment;
FIG. 16 is a block diagram of components for transmitting data between the medicine-taking management apparatus and a management server according to an embodiment;
FIG. 17 is a flowchart illustrating a process of a medicine-taking providing service according to an embodiment;
FIG. 18A is an exploded perspective view of a cutter according to an embodiment;
FIGS. 18B and 18C are front views illustrating an internal operation of the cutter according to an embodiment;
FIG. 19 is a perspective view of a storage unit according to another embodiment;
FIGS. 20 and 21 are cross-sectional views illustrating a successive dispensing operation of the storage unit of FIG. 19;
FIG. 22 is a perspective view of a medicine-taking management apparatus according to an embodiment;
FIG. 23 is an exploded perspective view illustrating a state in which a preservation unit is separated from the medicine-taking management apparatus of FIG. 22;
FIG. 24 is a perspective view illustrating a state in which the preservation unit is opened according to an embodiment;
FIG. 25 is a cross-sectional view illustrating the state in which the preservation unit is separated from the medicine-taking management apparatus of FIG. 22;
FIG. 26 is an exploded perspective view illustrating inner components of the medicine-taking management apparatus of FIG. 22;
FIG. 27 is a perspective view of an extension part and a path selection driving part according to an embodiment;
FIG. 28 is a partial perspective view illustrating operations of the extension part and the path selection driving part according to an embodiment;
FIGS. 29 and 30 are cross-sectional view of the extension part according to the operation state in FIG. 8;
FIGS. 31 to 36 are cross-sectional views illustrating an operation process of the medicine-taking management apparatus according to an embodiment; and
FIGS. 37 and 38 are cross-sectional views of a medicine-taking management apparatus including a preservation unit according to another embodiment.

### DETAILED DESCRIPTION OF THE INVENTION

Hereinafter, embodiments of the present invention will be described with reference to the accompanying drawings. If there is no specific definition or reference, the term representing a direction used in this description is on the basis of a state illustrated in the drawings. Also, the same reference numeral denotes the same member throughout embodiments. In the drawings, a thickness or size may be exaggerated for convenience of descriptions, but it does not mean that the thickness or size of each element does not entirely reflect an actual size.

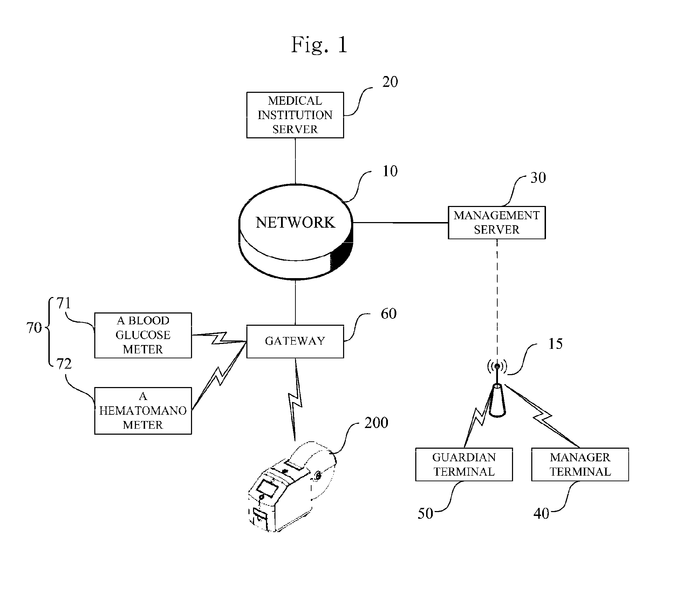

The whole structure of a medicine-taking management system and a connection relationship between respective components of the medicine-taking management system according to an embodiment of the present invention will be described with reference to FIG. 1. FIG. 1 is a block diagram illustrating the whole structure of a medicine-taking management system according to an embodiment of the present invention.

An external network 10 may include an Internet network in a broad sense. However, a physical configuration for establishing the network may be variously embodied. The external network 10 may be embodied by a public switched telephone network (PSTN), a near field communication network, infrared-ray communication, a mobile communication radio network, and at least one combination thereof. Hereinafter, the external network 10 may be distinguished from devices for connecting a medicine-taking management apparatus 100 and an internal network with respect to a gateway 60 and may not be limited in physical implementation method or position.

A medical institution server 20 is connected to a management server 30 and the external network 10. The medical institution server 20 may be a server that receives medicine-taking history information provided from the management server 30 to refer to in a medical practice process at the medical institution. The medical institution server 20 may be embodied as physically the same server as that in which data related to a medical treatment process, result, and prescription in the medical institution is stored and processed.

The management server 30 is connected to the medicine-taking management apparatus 100 and the medical institution server 20 through the network. The management server 30 receives the medicine-taking history information from the medicine-taking management apparatus 100 to transmit the received medicine-taking history information to the medical institution server 20. Also, if an additional medicine-taking history management is required, the management server 30 may transmit various types of data to a guardian terminal 50, a manager terminal 40, and the like through a mobile communication base station 15 or the external network 10. Specific functions with respect to the management server 30 will be described in detail with reference to related parts.

The guardian terminal 50 represents a terminal of a patient to be managed according to a medicine-taking history thereof. That is, the guardian terminal 50 may include the patients' own terminal as well as the guardian terminal 50. Various types of terminals such as computing devices, for example, a mobile phone, a smart phone, a notebook, and the like may be used as the guardian terminal 50. The guardian terminal 50 may receive data from the management server 30 and/or the medicine-taking management apparatus 100 through various types of communication networks such as the mobile communication base state 15, the external network 10, and the like.

The manager terminal 40 represents a medicine-taking management service providing-side terminal. Also, various types of terminals such as computing devices, for example, a mobile phone, a smart phone, a notebook, and the like may be used as the manager terminal 40. The guardian terminal 50 may receive data from the management server 30 and/or the medicine-taking management apparatus 100 through various types of communication networks such as the mobile communication base state 15, the external network 10, and the like. Alternatively, the guardian terminal 50 may receive data from the internal network provided on a side of the management server 30.

The gateway 60 may be a device for forming an internal network in a high-rank network of the medicine-taking management apparatus 100 and transmitting/receiving data between the medicine-taking management apparatus 100 or a biometric information measuring instrument 70 which is connected to low-rank units of the gateway 60 and the external network 10. The gate way 60 may transmit/receive data into/from the low-rank units thereof in a wireless or wired manner.

The biometric information measuring instrument 70 represents a device for measuring patient own biometric information. The biometric information measuring instrument 70 may include devices for measuring various biometric information such as a blood glucose meter 71, a hematomanometer 72, a digital thermometer, and the like. The biometric information measuring instrument 70 may be wiredly connected to the gateway 60 to transmit/receive data. Alternatively, the biometric information measuring instrument 70 may be connected to the gateway 60 by using a near field wireless network such as a Bluetooth communication module. The various biometric information measured by the biometric information measuring instrument 70 may be transmitted into the medicine-taking management apparatus 100 through the gateway 60.

A prescribed chartula package may be mounted on the medicine-taking management apparatus 100. Also, the medicine-taking management apparatus 100 may help the medicine-taking of the patient according to a prescribed medicine-taking period and chartula package information and collect related data. The medicine-taking management apparatus 100 may receive biometric information from the various biometric information measuring instruments 70 through the gateway 60 to collect medicine-taking history information, thereby transmitting the collected medicine-taking history information to the management server 30. As occasion demands, the medicine-taking management apparatus 100 may perform management functions according to the medicine-taking history.

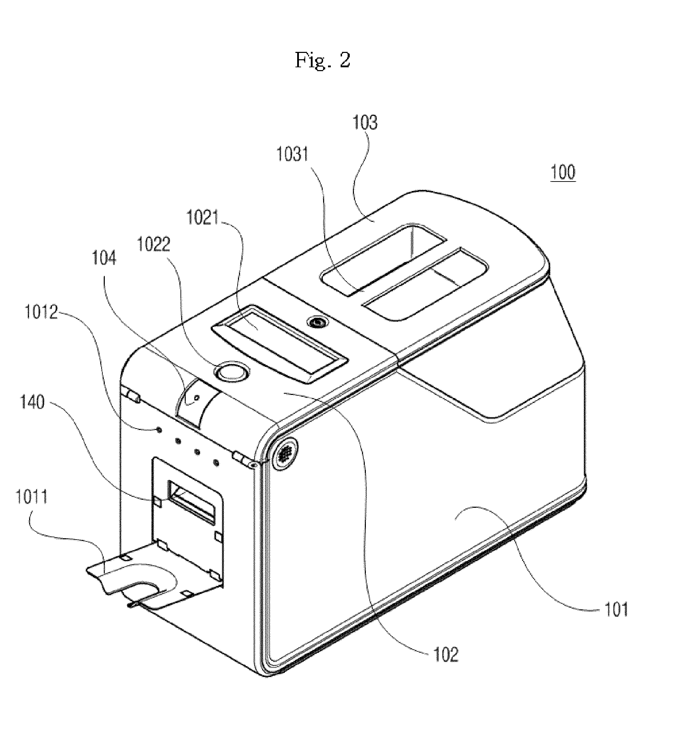

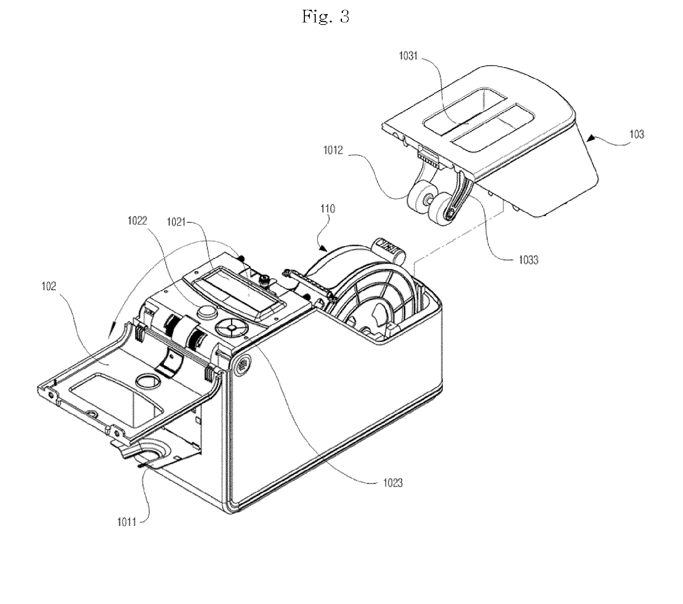

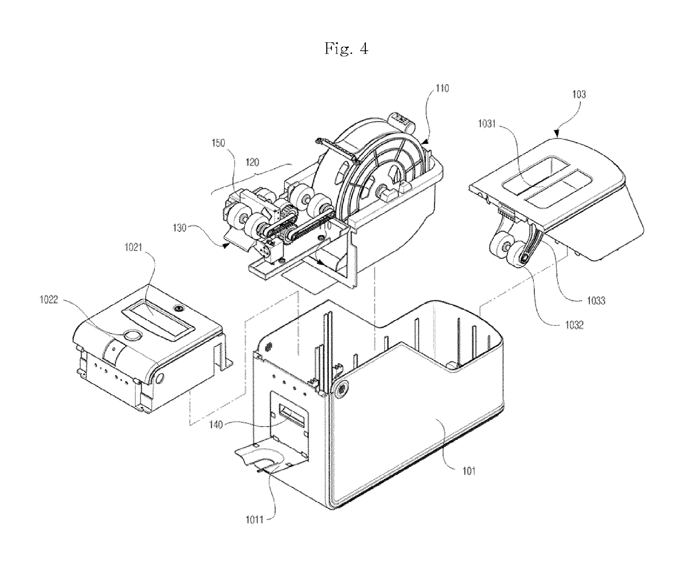

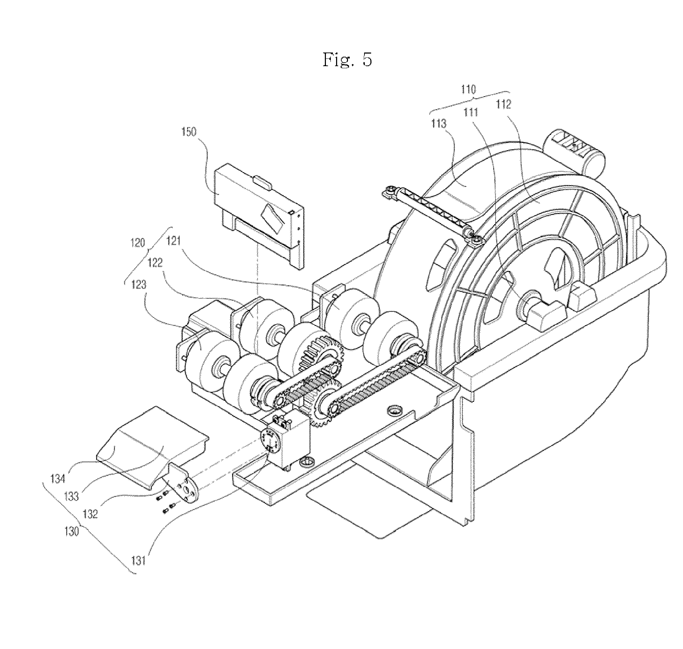

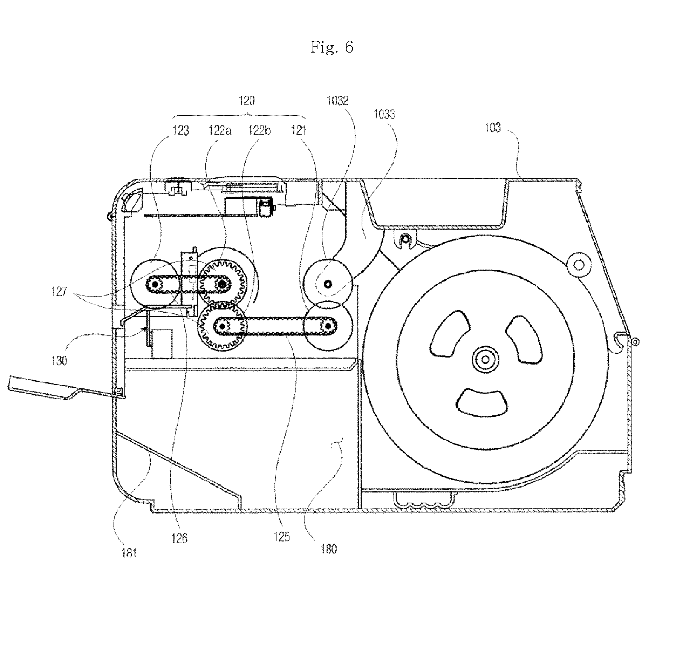

Hereinafter, components related to the transfer of the chartula package in the medicine-taking management apparatus 100 will be described in detail with reference to FIGS. 2 to 6. FIG. 2 is a perspective view of the medicine-taking management apparatus according to an embodiment, FIG. 3 is a perspective view of a state in which an upper case of the medicine-taking management apparatus is separated according to an embodiment, and FIG. 4 is an exploded perspective view of the medicine-taking management apparatus according to an embodiment. Also, FIG. 5 is an exploded perspective view illustrating main components of the medicine-taking management apparatus according to an embodiment, and FIG. 6 is a cross-sectional view of the medicine-taking management apparatus according to an embodiment.

Referring to FIG. 2, a housing 101, a protection cover 102, and an upper cover 103 are provided on an outer portion of the medicine-taking management apparatus 100. The housing 101 surrounds side surfaces and a bottom surface of the medicine-taking management apparatus 100. The protection cover 102 and the upper cover 103 cover an upper portion of the medicine-taking management apparatus 100. Referring to FIG. 3, the protection cover 102 has an edge rotatably coupled to the housing 101. The protection cover 102 may open or close an upper end of the housing 101 and restrain approach of a setting button 1023 disposed on the upper end of the housing 101. A dispensing button 1022 may be exposed to the outside through the protection cover 102. The dispensing button 1022 may operate the medicine-taking management apparatus 100 to dispense a chartula according to patient's input.

A dispensing hole 140 through which the chartula is dispensed is defined in a front surface of the housing 101. Also, a seating part 1011 on which the dispensed chartula is seated is provided under the dispensing hole 140. A display part 1012 is disposed above the dispensing hole 140. The display part 1012 may include a plurality of LEDs to emit light in various manners through the LEDs according to an operation state of the medicine-taking management apparatus 100 to allow the patient to recognize the operation state of the medicine-taking management apparatus 100.

An imaging part 104 is disposed on an upper end of the front surface of the housing 101. The imaging part 104 may photograph a surrounding image of the dispensing hole 140. An operation method and operation time of the imaging part 104 will be described below in detail.

The upper cover 103 is disposed on the upper portion of the housing 101. As shown in FIG. 3, the upper cover 103 covers a portion of a top surface of the housing 101 except for the portion covered by the protection cover 102. A pair of connection arms 1033 extend downward from the inside of a top surface of the upper cover 103. A support roll 1012 is disposed on an end of each of the connection arms 1033.

A preservation unit 110 provided within the housing 101 is exposed in a state where the upper cover 103 is opened. The preservation unit 110 may be a component on which the prescribed chartula package is initially mounted.

As shown in FIG. 4, main components for dispensing the chartula package may include the preservation unit 110, a transfer unit 120, a cutting unit 150, and a path selection unit 130. The transfer unit 120 is disposed adjacent to the preservation unit 110 and disposed between the cutting unit 150 and the transfer unit 120. The path selection unit 130 is disposed on the other side of the preservation unit 110 with respect to the transfer unit 120, i.e., disposed adjacent to the dispensing hole 140.

Referring to FIG. 5, the preservation unit 110 includes core 111, a side surface case 112, and a separation prevention part 113. The preservation unit 110 may have a wheel shape including the core 111 and a pair of side surface cases 112 each constituted by a circular plate around the core 111. The core 111 may serve as a core around which the chartula package is wound. The side surface cases 112 may prevent the chartula package from being laterally separated. The separation prevention part 113 may have a curved shape to cover a space part between both side surface cases 112. The separation prevention part 113 may prevent the chartula package from being radially separated from a center of the core 111 after the chartula package is wound around the core 111.

Referring to FIGS. 4 and 5, the path selection unit 130 includes a rotation shaft 131, a connection arm 132, a support plate 133, and an inclination part 134. The path selection unit 130 includes the rotation shaft 131 that is rotated about an axis defined by a transfer direction of the chartula package, i.e., a direction in which rolls of the transfer unit 120 are successively arranged. The connection arm 132 extends toward a lower portion of a discharge roll 123 of the transfer unit 120 from the rotation shaft 131. The support plate 133 is disposed on each of an end of the connection arm 132 and the lower portion of the discharge roll 123. The support plate 133 may contact a lower end of the discharge roll 123. The inclination part 134 is disposed on an end of the support plate 133, i.e., an outlet side of the support plate 133 in the transfer direction of the chartula package. An outlet side of the path selection unit 130 may be disposed adjacent to the above-described dispensing hole 140 to form a continuous transfer path of the chartula package.

As shown in FIG. 5, the cutting unit 150 is disposed in a chartula package transfer path of the transfer unit 120. Particularly, the cutting unit 150 is disposed between the discharge roll 123 and a driving roll 122 to cut the foremost chartula of the chartula package.

Referring to FIGS. 5 and 6, the transfer unit 120 includes an unwinding roll 121, the driving roll 122, and the discharge roll 123. The unwinding roll 121 is disposed on an inlet side in the transfer path of the transfer unit 120. The chartula package may be mounted in a state where the foremost chartula is placed on the unwinding roll 121. As the unwinding roll 121 is rotated, the chartula placed on the unwinding roll 121 is transferred. A pair of driving rolls 122a and 122b are disposed on an outlet side of the unwinding roll 121. The driving rolls 122a and 122b are vertically disposed. Also, the driving rolls 122a and 122b may be mutually engaged with each other through a gear. Thus, when torque is transferred to one driving roll of the upper and lower driving rolls 122a and 122b through a motor, the other driving roll may be also rotated. The discharge roll 123 is disposed on an outlet side of the driving rolls 122a and 122b. The discharge roll 123 is disposed on the rearmost end of the transfer unit 120 to discharge the cut chartula. A support roll 1032 that is rotatably fixed to the connection arm 1033 extending downward from the top surface of the upper cover 103 may be disposed above the unwinding roll 121. The support roll 1032 may support the chartula disposed between the support roll 1032 and the unwinding roll 121 from an upper side to easily unwind and transfer the chartula.

The upper and lower driving rolls 122a and 122b may be engaged with each other the gear so that the torque is transmitted therebetween. The discharge roll 123 and the upper driving roll 122a, and the lower driving roll 122b and the unwinding roll 121 may be respectively connected to each other by using timing belts 126 and 125 to transmit the torque therebetween. Separate torque may not be transmitted to the support roll 1032.

An outer surface of each of the rolls 121, 122, and 123 of the transfer unit 120 may be formed of a porous elastic material such as sponge to prevent the rolls 121, 122, and 123 from pressing the chartula.

A storage unit 180 may be provided as a predetermined space part which is defined under the path selection unit 130 and the transfer unit 120 to store the chartula. Also, an inclined guide part 181 is disposed under the path selection unit 130 so that the chartula is seated when the chartula drops.

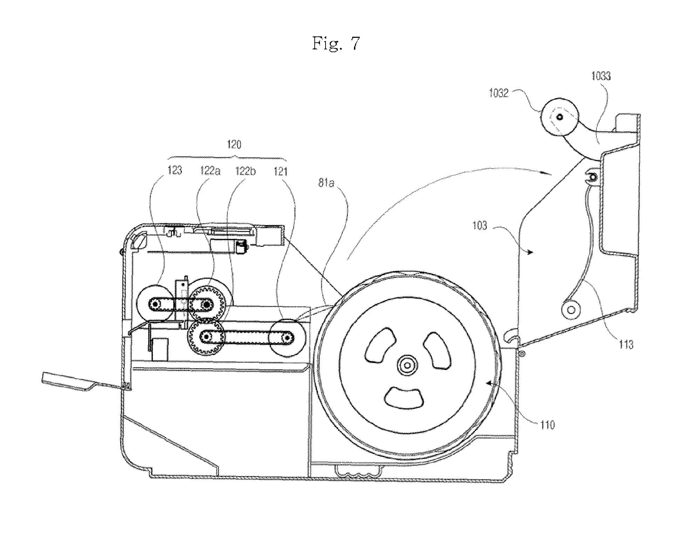

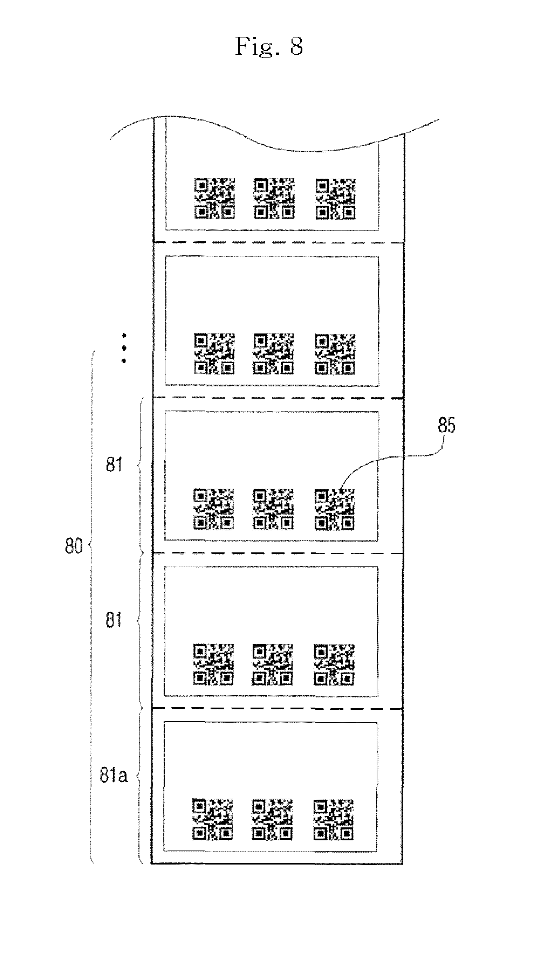

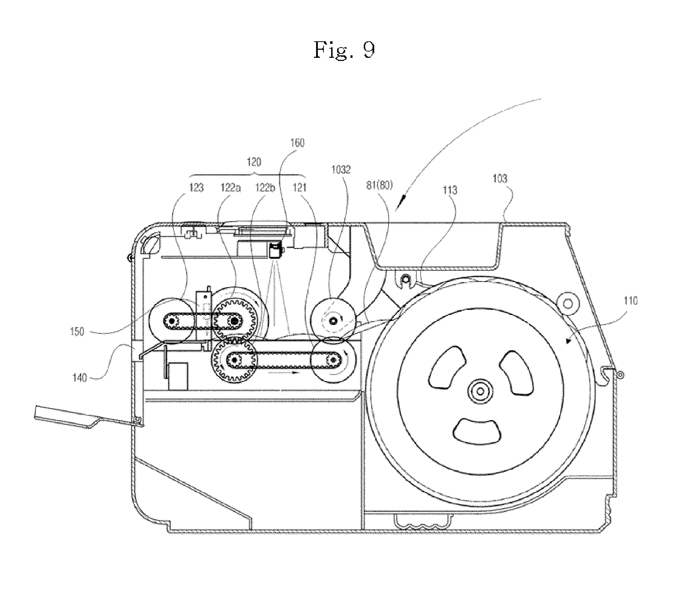

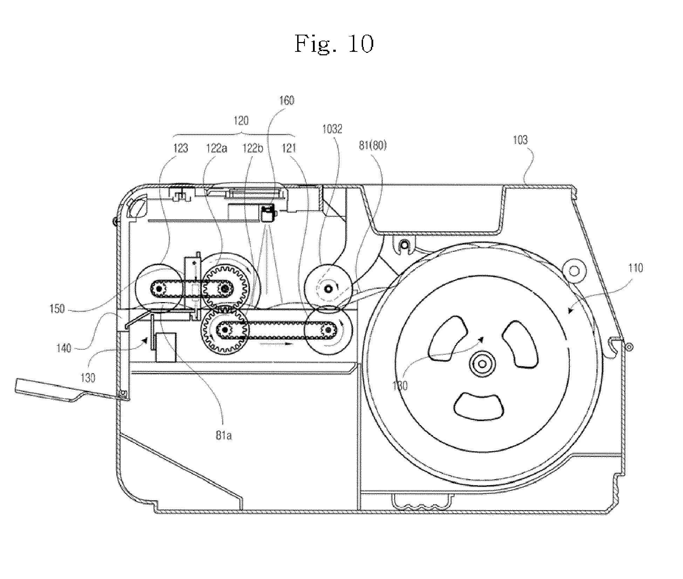

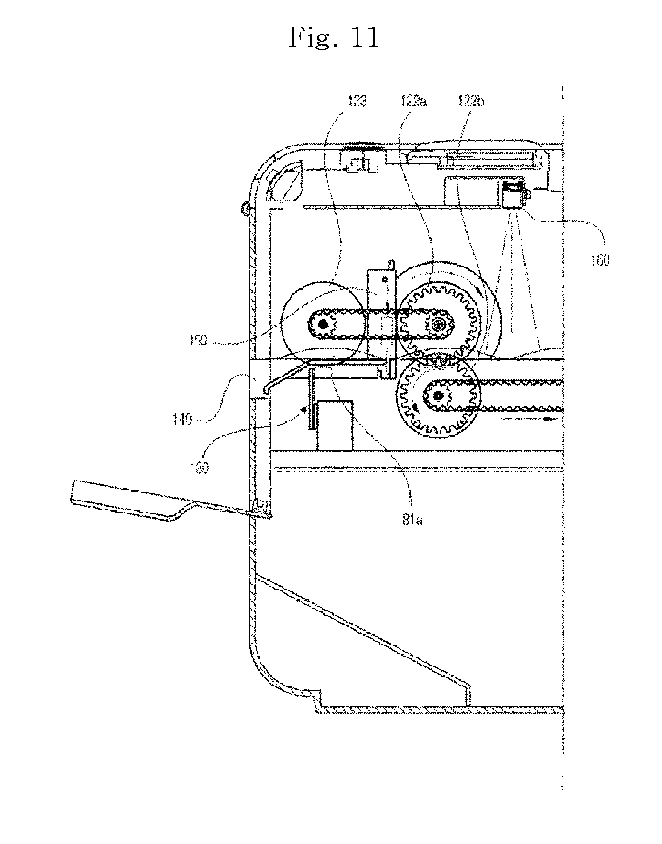

Processes for mounting and transferring the chartula by using the medicine-taking management apparatus together with the components according to an embodiment will be described in detail with reference to FIGS. 7 to 11. FIG. 7 is a cross-sectional view illustrating an initial process for mounting the chartula package on the medicine-taking management apparatus according to an embodiment, and FIG. 8 is a schematic view of the chartula package according to an embodiment. Also, FIG. 9 is a cross-sectional view illustrating a state in which the chartula package is transferred within the medicine-taking management apparatus according to an embodiment, FIG. 10 is a cross-sectional view illustrating a state in which a portion of the chartula package is cut within the medicine-taking management apparatus according to an embodiment, and FIG. 11 is a partially enlarged cross-sectional view illustrating a state in which the portion of the chartula package of FIG. 10 is cut.

Referring to FIG. 7, the chartula package is wound around on the preservation unit 110 in the state where the upper cover 103 is opened. Here, the foremost chartula of the chartula package is disposed on an upper end of the unwinding roll 121.

For example, a chartula package 80 may be arranged in a shape as shown in FIG. 8. That is, the chartula package 80 may include a plurality of chartulas 81 that are continuously connected to each other. A chartula of the series of chartula package 80 which is disposed on the upper end of the unwinding roll 121 may be defined as the foremost chartula 81a. In the chartula package 80 wound around the preservation unit 110, the foremost chartula 81a may be first transferred when the chartula is transferred for dispensing. Then, the foremost chartula 81a may be cut and dispensed, and then, a chartula disposed adjacent to the foremost chartula 80a may become to the foremost chartula 81a newly.

A two-dimensional graphic code, for example, a QR code may be printed on each of the chartulas 81 of the chartula package 80. The QR code may include medicine-taking information such as patient's identification data, a medicine-taking time, chartula package information, and the like. Here, the patient's identification data may include a series of number for identifying a patient that is an object for taking a medicine or patient's personal data. The medicine-taking time and the chartula package information may include information related to a kind of medicine and a time for taking a medicine.

Next, as shown in FIG. 9, the upper cover 103 is covered. Here, the support roll 1032 connected to a lower portion of the top surface of the upper cover 103 may contact the upper portion of the unwinding roll 121 to support the chartula 81 and the chartula package 80, thereby preventing the chartula 81 and the chartula package 80 from being separated.

Then, when the driving rolls 122a and 122b are rotated, the unwinding roll 121 may be rotated together with the driving rolls 122a and 122b to transfer the chartula package 80 disposed between the unwinding roll 121 and the support roll 1032 toward the dispensing hole 140.

A scan part 160 is disposed in the transfer path of the chartula package 80. The scan part 160 may be disposed between the driving roll 122 and the unwinding roll 121 in the transfer path. The scan part 160 scans the QR code printed on the chartula package 80 and a cutoff position or dotted line of the chartula package 80. The scan part 160 may be embodied in various manners. That is, a scanner for scanning the two-dimensional graphic code and the dotted line may be separately provided. Alternatively, the two-dimensional graphic code and the dotted line may be photographed and recognized at the same time by using a camera.

Thereafter, when the chartula is dispensed or stored, as shown in FIGS. 10 and 11, the cutting unit 150 may be operated to cut or separate the foremost chartula 81a in a state where the foremost chartula 81a is disposed between the discharge roll 123 and the path selection unit 130.

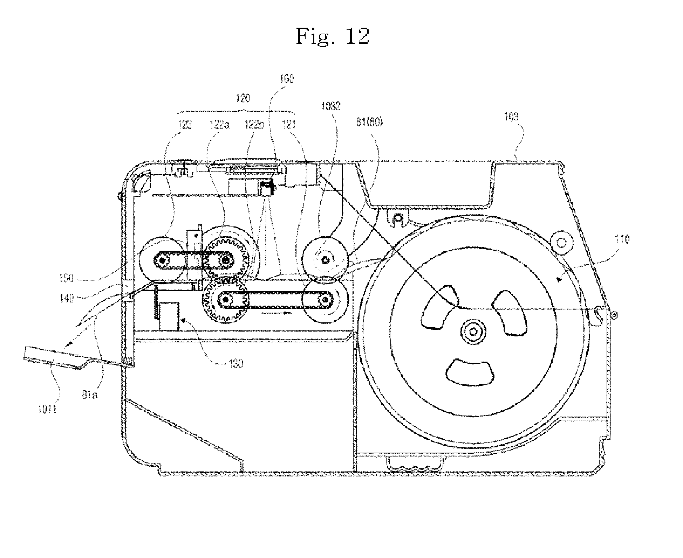

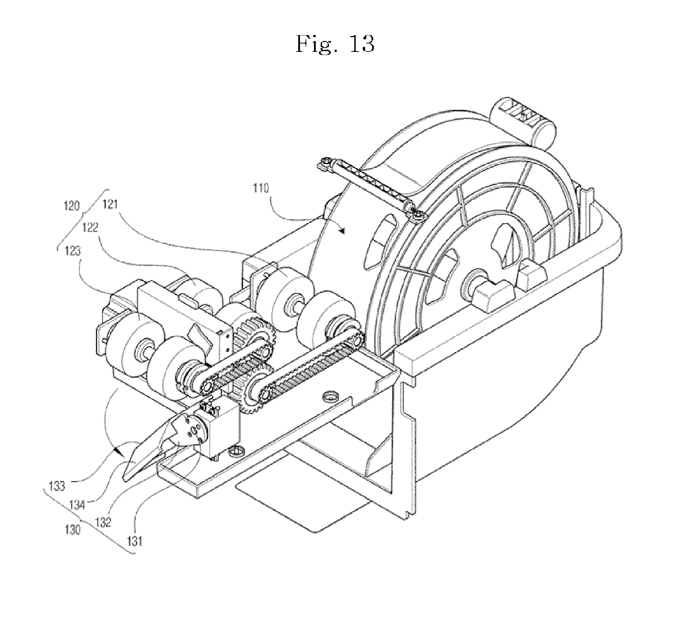

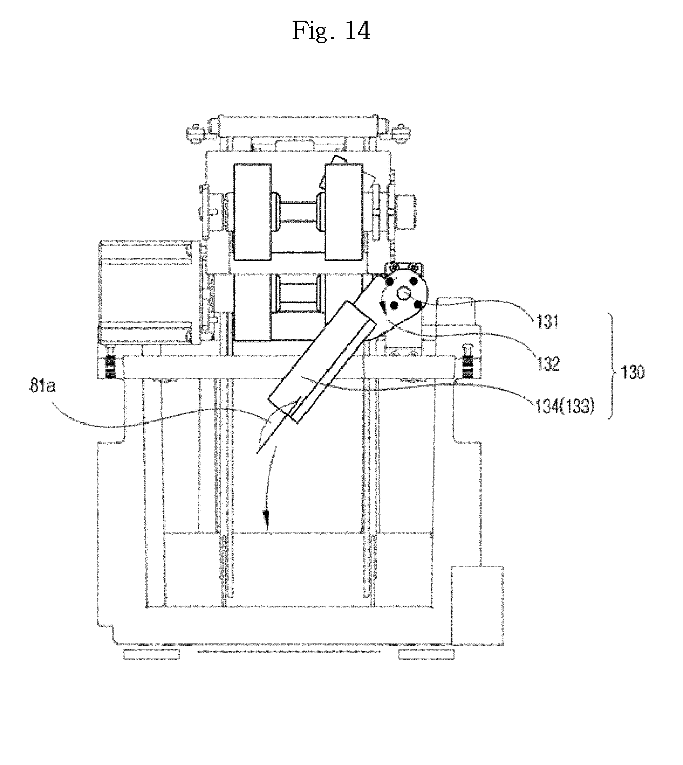

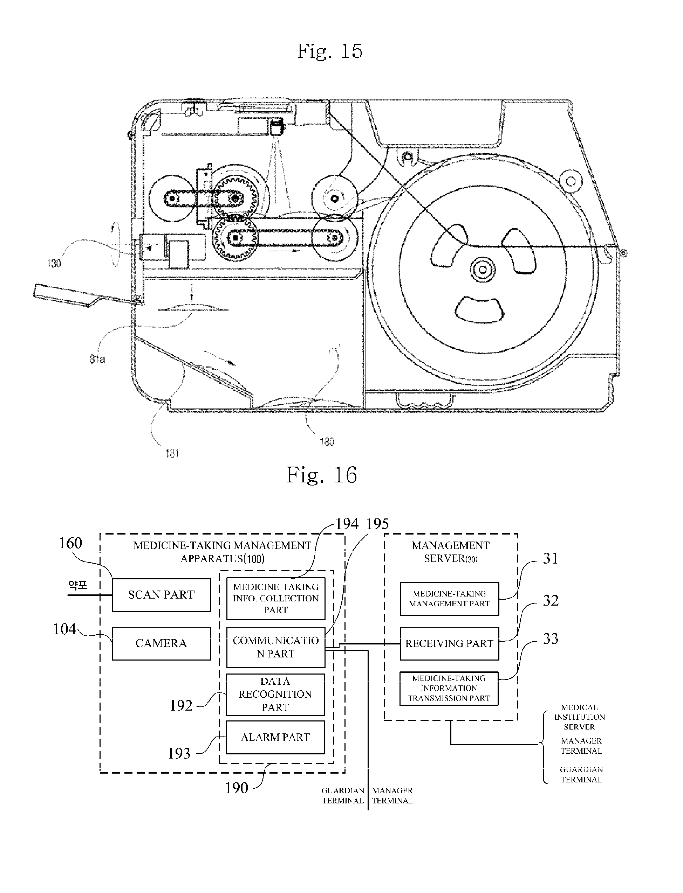

Thereafter, the foremost chartula 81a may be moved along two paths according to an operation state of the path selection unit 130. A process of dispensing or storing the cut chartula through the two paths will be described with reference to FIGS. 12 to 15. FIG. 12 is a cross-sectional view illustrating a state in which a chartula is dispensed through a first path of the medicine-taking management apparatus according to an embodiment, and FIG. 13 is a partial perspective view illustrating an operation process for selecting a second path within the medicine-taking management apparatus according to an embodiment. Also, FIG. 14 is a partial front view illustrating an operation process for selecting the second path within the medicine-taking management apparatus according to an embodiment, and FIG. 15 is a cross-sectional view illustrating a state in which a chartula is stored through the second path within the medicine-taking management apparatus according to an embodiment.

The first path represents a path through which the chartula is dispensed to the outside of the device, and the second path represents a path through which the chartula is stored into a separate space within the device.

In detail, as shown in FIG. 12, the first path may be a path through which the foremost separated chartula 81a is dispensed through the dispensing hole 140. In this case, the path selection unit 130 may be in contact with the discharge roll 123. Also, as described above, the outlet-side end of the path selection unit 130 may form a path toward the dispensing hole 140. As the discharge roll 123 is rolled, the foremost chartula 81a may be dispensed through the dispensing hole 140.

On the other hand, when the second path is selected as shown in FIGS. 13 and 14, the rotation shaft 131 of the path selection unit 130 may be rotated to allow each of the support plate 133 and the inclination part 134 to form a downward inclination. In this case, the foremost separated chartula 81a disposed on the support plate 133 may be slid along the downward inclination of the support plate 133. As a result, as shown in FIG. 15, the foremost chartula 81a may drop onto the support plate 133 to drop into the storage unit 180. The foremost dropping chartula 81a may contact the guide part 181 firstly and then be slid along the inclination of the guide part 181, and thus be collected onto the lowermost end of the storage unit 180.

Components related to a flow of data of the medicine-taking management system according to the present invention will be described with reference to FIG. 16. FIG. 16 is a block diagram of components for transmitting data between the medicine-taking management apparatus and a management server according to an embodiment.

The data transmission of the medicine-taking system according to the present invention may occur with respect to a medicine-taking management device and the management server 30. Hereinafter, components of the medicine-taking management apparatus 100 and the management server 30 which are related to the data transmission will be described in detail.

The medicine-taking management apparatus 100 includes a scan part 160, the imaging part 104, and a control part 190. As described above, the scan part 160 may scan the QR code printed on the chartula and the dotted line. The imaging part 104 may photograph a surrounding still image or moving image of the medicine-taking management apparatus 100, i.e., a front still or moving image of the medicine-taking management apparatus 100.

The control part 190 controls a roller within the medicine-taking management apparatus 100 or the motor for rotating the rotation shaft. Also, the control part 190 may receive data from the scan part 160 to separate the foremost chartula. The control part 190 may control the imaging part 104 so that the surrounding image of the medicine-taking apparatus 100 at a specific time at which the chartula is dispensed. In detail, the imaging part 104 photographs a moving or still image that is capable of being used for determining whether the patient takes the medicine. For example, the imaging part 104 may photograph a front side of the medicine-taking apparatus 100 when the medicine-taking apparatus 100 is operated to photograph a person dispensing the chartula from the medicine-taking apparatus 100 or photograph a side of the dispensing hole 140 to photograph an image for confirming whether the chartula is actually dispensed. Also, the imaging part 104 is not limited in photographing time or period. That is, the imaging part 104 may be used for acquiring multimedia data as auxiliary data for determining whether the patient takes the medicine by the manager or guardian.

In addition, the control unit 190 may further include a medicine-taking information collection part 104, a communication part 195, a data recognition part 192, and an alarm part 193.

The medicine-taking information collection part 194 may determine whether the patient takes the medicine such as whether the chartula is normally dispensed according to the medicine-taking time or whether the medicine is stored in the storage unit after the medicine-taking time elapses. Also, the medicine-taking information collection part 194 may collect medicine-taking history information including the normal medicine-taking history and medicine-untaking information in addition to whether the medicine is normally taken into the patient. Here, the medicine-untaking information may include a non-dispensing number of a medicine and non-dispensed chartula information. Also, the medicine-taking information collection part 194 may compare the medicine-taking information to the medicine-taking history information which are obtained from data scanned by the scan part 160.

The communication part 195 transmits/receives data into/from the management server 30. For example, the communication part 195 may transmit various medicine-taking history information collected by the medicine-taking information collection part 194 or the still or moving image photographed by the imaging part 194. Also, the communication part 195 may transmit the patient's biometric information and the result obtained by comparing the medicine-taking information to the medicine-taking history information by using the medicine-taking information collection part 194 to the management server 30. The data may be transmitted in various manners by the communication part 195. For example, in the case of the medicine-taking history information, the data may be periodically transmitted to he management server 30 or transmitted to the management server 30 only when the chartula is not actually dispensed.

The communication part 195 may transmit the medicine-taking history information, particularly, the medicine-untaking information to the guardian terminal or the manager terminal. In this case, the above-described information may be transmitted in various forms such as an SMS, a mail, an audio guidance, or an audio message.

The data recognition part 192 may recognize medicine-taking information such as the patient identification data, the medicine-taking time, and the chartula package information from the data scanned by the scan part 160. The alarm part 193 may generate an alarm for informing that the medicine-taking time reaches to the patient corresponding to the medicine-taking time of the medicine-taking information.

The management server 30 includes a medicine-taking management part 31, a receiving part 32, and a medicine-taking information transmission part 33. The receiving part 32 receives various information together with the medicine-taking history information transmitted from the communication part 195 of the medicine-taking management apparatus 100. The medicine-taking information transmission part 33 may transmit the patient's medicine-taking history to the medical institution server 20.

The medicine-taking management part 31 may perform a follow-up management function when the medicine-untaking information is included in the received medicine-taking history information. The medicine-untaking information represents information through which it is analogized that the chartula is not normally dispensed at the normal medicine-taking time, such as the non-dispensing number of the medicine and the non-dispensed chartula information. The medicine-taking management part 31 may transmit the corresponding information to the manager terminal or guardian terminal when the medicine-untaking information is included in the medicine-taking history information.

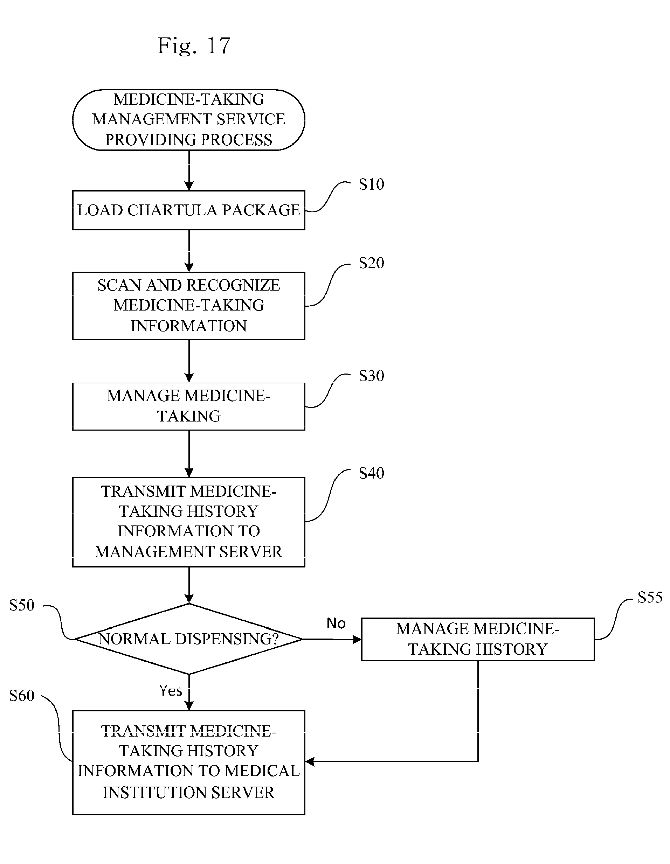

A process of a medicine-taking providing service will be described with reference to FIG. 17. FIG. 17 is a flowchart illustrating a process of a medicine-taking providing service according to an embodiment.

First, a chartula package is loaded (S10). As described above, the chartula package is mounted within the medicine-taking management apparatus. Then, the medicine-taking management apparatus may scan and recognize a QR code printed on the chartula package to load the chartula package. The loading information may also be transmitted from the medicine-taking management apparatus to the management server.

Next, the medicine-taking management apparatus may scan a two-dimensional graphic code printed on the chartula package to recognize a medicine-taking time and chartula package information (S20).

Then, the medicine-taking management apparatus provides an alarm to a patient according to the recognized medicine-taking information, thereby providing the chartula to the patient (S30). However, when the corresponding chartula is not dispensed at the medicine-taking time, the non-dispensed corresponding chartula may be separated from the chartula package to separately store the separated chartula package into the storage unit. In this process, the medicine-taking management apparatus may collect medicine-taking history information such as a kind of chartula, a providing time, medicine-untaking number, medicine-untaking time, and a kind of medicine-untaking chartula.

Then, the medicine-taking history information is transmitted into the management server at a preset time (S40). Here, as described above, the medicine-taking history information may be periodically transmitted or transmitted only when the medicine-untaking information is collected.

Then, the management server may determine whether the transmitted medicine-taking history information includes the medicine-untaking information (S50). If the transmitted medicine-taking history information does not include the medicine-untaking information, the corresponding medicine-taking history information is transmitted the medical institution server (S60). On the other hand, if the transmitted medicine-taking history information includes the medicine-untaking information, the medicine-taking management is performed before or after the corresponding medicine-taking history information is transmitted the medical institution server (S55). Here, as described above, the medicine-taking history management may represent the follow-up management in which the medicine-untaking information is transmitted to the manager terminal or guardian terminal.

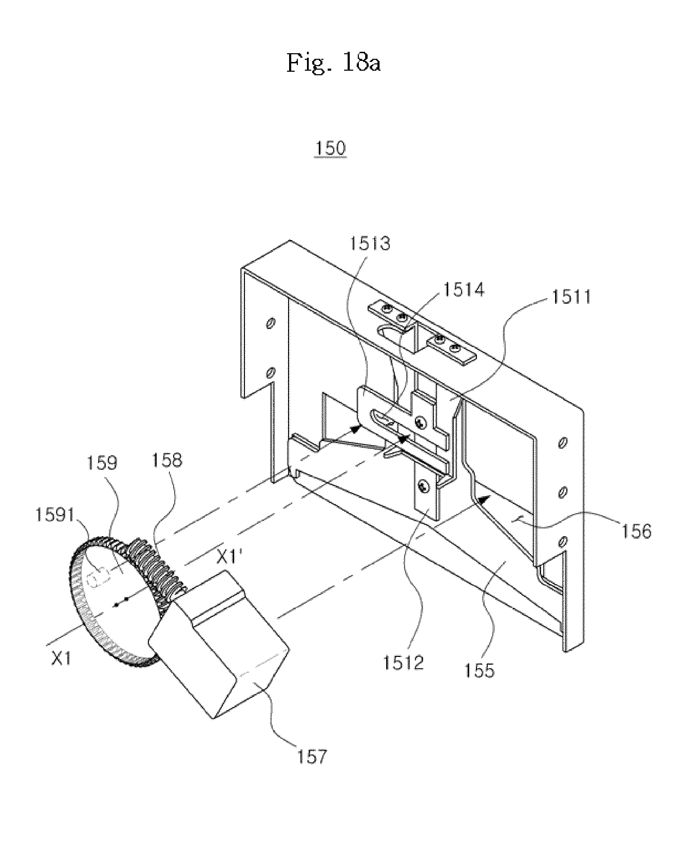

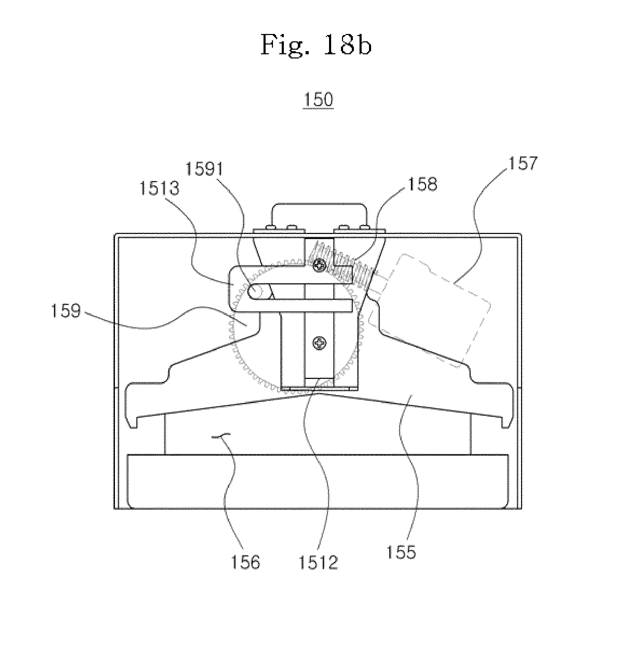

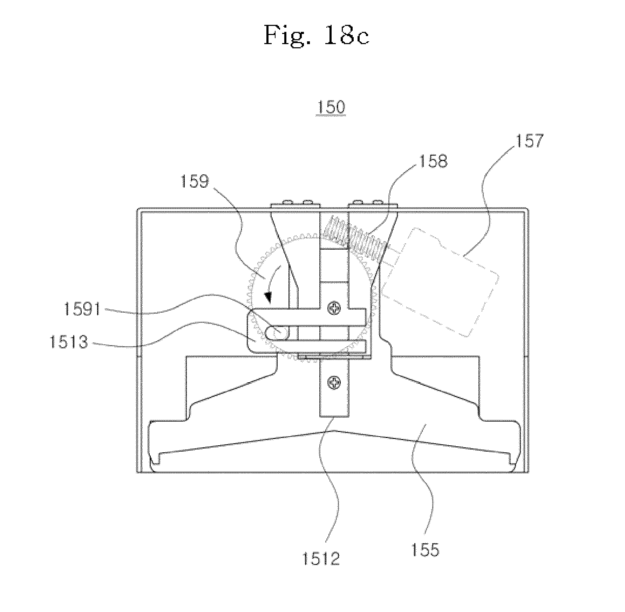

A cutter 150 will be described with reference to FIGS. 18A to 18C. FIG. 18A is an exploded perspective view of a cutter according to an embodiment, and FIGS. 18B and 18C are front views illustrating an internal operation of the cutter according to an embodiment.

Referring to FIG. 18A, the cutter 150 includes a chartula moving hole 156 through which a chartula passes and a blade 155 for cutting the chartula transferred through the chartula moving hole 156. The blade 155 is fixed to an elevation part 1512 and an extension part 1513. The elevation part 1512 is reciprocated along an elevation guide part 1511 in an operation direction of the blade 155. The extension part 1513 is fixed to the elevation part 1512 to move together with the elevation part 1512. The extension part 1513 has a groove 1514 in one side thereof. A protrusion 1592 disposed on a first gear part 159 is inserted into the groove 1514. The first gear part 159 receives rotation force of a motor 157 through a second gear part 158 to rotate. In detail, when the motor 157 is rotated, the second gear part 158 rotated about the same shaft as the motor 157 and having a screw thread on an outer circumference surface thereof is rotated together. When the second gear part 158 is rotated, the first gear part 159 engaged with the second gear part 158 is rotated together.

Referring to FIGS. 18B and 18C, when the first gear part 158 is rotated, the protrusion 1591 is rotated together. When the protrusion 1591 is rotated to descend, the extension part 1513 in which the protrusion 1592 is inserted descends. Thus, the blade 155 fixed to the extension part 1513 descends together. When the blade 155 descends, the chartula transmitted through the chartula moving hole 156 is cut.

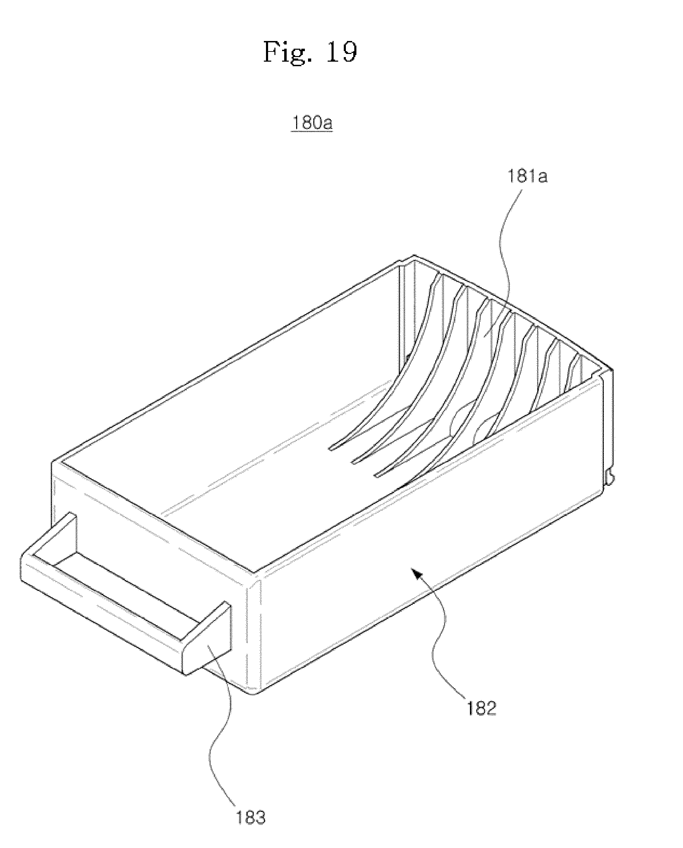

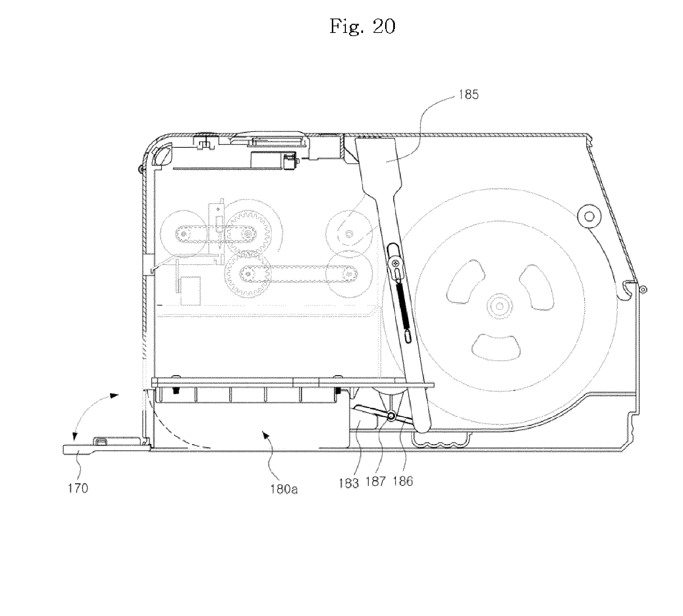

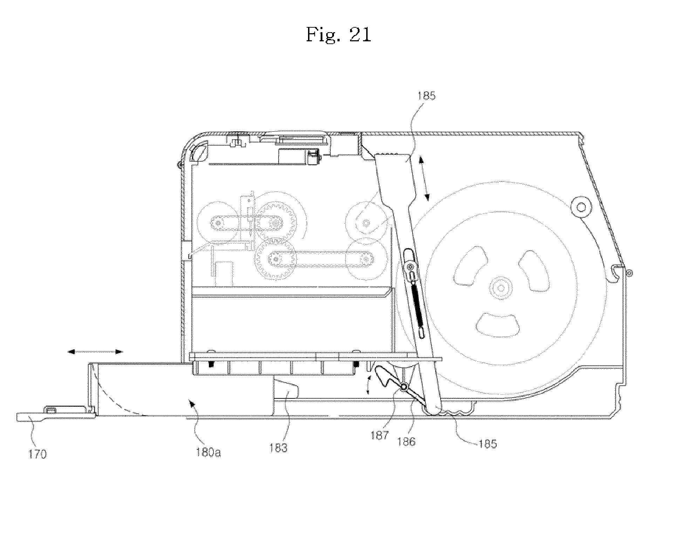

A storage part according to another embodiment will be described with reference to FIGS. 19 to 21. FIG. 19 is a perspective view of a storage unit according to another embodiment, and FIGS. 20 and 21 are cross-sectional views illustrating a successive dispensing operation of the storage unit of FIG. 19.

Referring to FIG. 19, a storage unit 180a according to the current embodiment may have a predetermined space in which a chartula is stored in a drawer-type space. Also, an inclined guide part 181a is disposed within the storage unit 180a so that the dropping chartula is pushed and stacked to one side.

Also, in the storage unit 180a, a hanging part 183 is disposed on the outer side of the guide part 181a. The hanging part 183 extends from the storage unit 180a to protrude upward from an end thereof. The hanging part 183 may be hung on or released from a hook part 186 having a hook shape to fix or withdraw the storage unit 180a.

Referring to FIGS. 20 and 21, a lever 185, the hook part 186, a rotation shaft 187 are provided within the medicine-taking management apparatus. The hook part 186 has an end with a hook shape and is moved with a reciprocating angular motion with respect to the rotation shaft 187. The lever 185 may be operated to push or pull the other end of the hook part 186 so that a hook provided on one end of the hook part 186 is vertically moved.

When the lever 185 is pushed, the hook disposed on the one end of the hook part 186 ascends as shown in FIG. 21. Thus, the storage unit 180a may be released to freely withdraw the medicine-taking management apparatus to the outside.

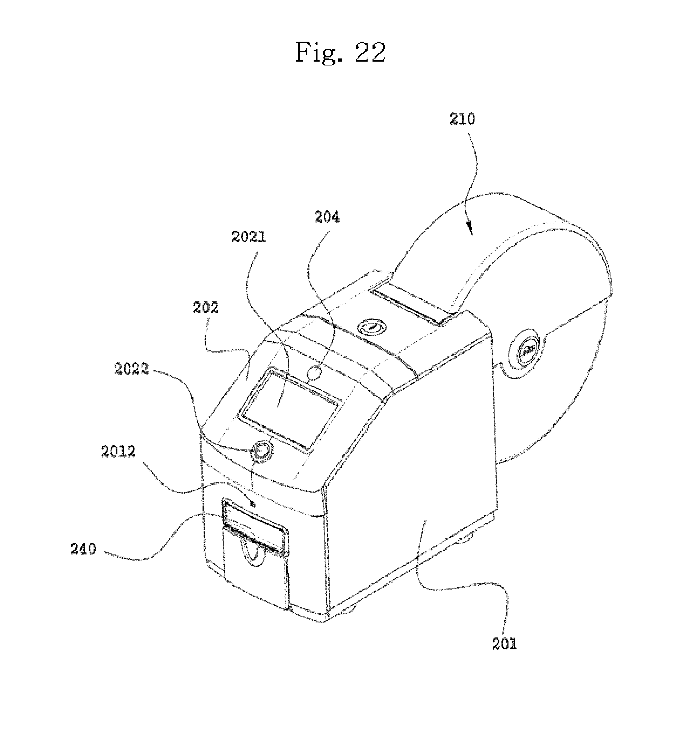

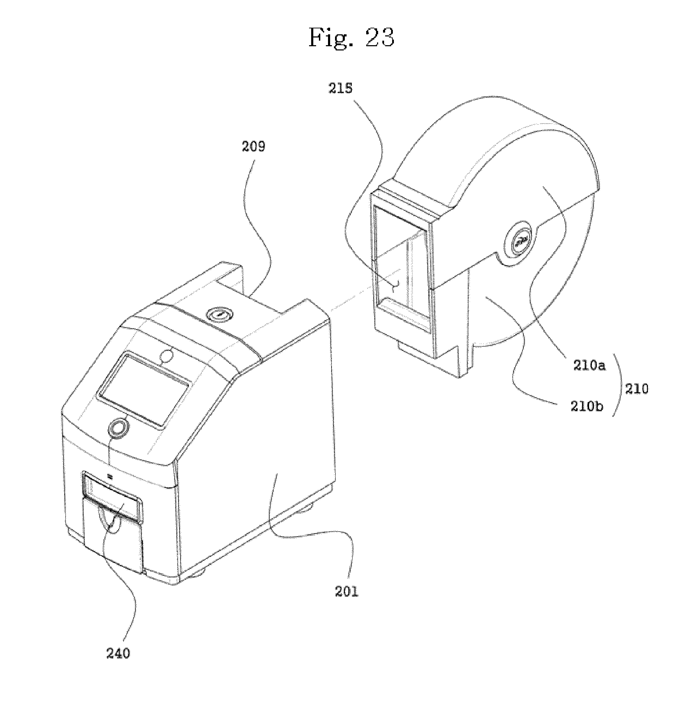

A medicine-taking management apparatus 200 according to another embodiment will be described with reference to FISG. 22 and 23. FIG. 22 is a perspective view of a medicine-taking management apparatus according to an embodiment, and FIG. 23 is an exploded perspective view illustrating a state in which a preservation unit is separated from the medicine-taking management apparatus of FIG. 22.

Referring to FIG. 22, a housing 201, a cover unit 202, and a preservation unit 210 are provided on an outer portion of the medicine-taking management apparatus 200. The housing 201 surrounds side surfaces and a bottom surface of the medicine-taking management apparatus 200. The cover unit 202 covers a front surface of an upper portion of the medicine-taking management apparatus 200. The cover unit 202 may provide a physical protection function for various setting buttons provided inside the cover part 202. A dispensing button 2022 may be exposed to the outside through the cover unit 202. The dispensing button 2022 may operate the medicine-taking management apparatus 200 to dispense a chartula according to patient's input.

A dispensing hole 240 through which the chartula is dispensed is defined in a front surface of the housing 201. A display part 2012 is disposed above the dispensing hole 240. The display part 2012 may include at least on LED to emit light in various manners through the LED according to an operation state of the medicine-taking management apparatus 200 to allow the patient to recognize the operation state of the medicine-taking management apparatus 200.

A display 2021 is disposed at a central portion of the cover unit 202. Information related to medicine-taking management and history may be displayed through the display 2021 to inform the medicine-taking management and history to a user.

An imaging part 204 is disposed on an upper end of the front surface of the housing 201. The imaging part 204 may photograph a surrounding image of the dispensing hole 240. An operation method and operation time of the imaging part 204 will be described below in detail.

The preservation unit 210 is disposed on a rear surface of the housing 201. The preservation unit 210 may be a component on which a chartula package is stored. The preservation unit 210 according to the current embodiment may have a structure that is detachable from the housing 201. Also, the preservation unit 210 according to the current embodiment may have an outer appearance defined by a first housing member 210b and a second housing member 210b. An opening 215 that is a passage through which the chartula package preserved within the preservation unit 210 is withdrawn is defined in one side of the preservation unit 210.

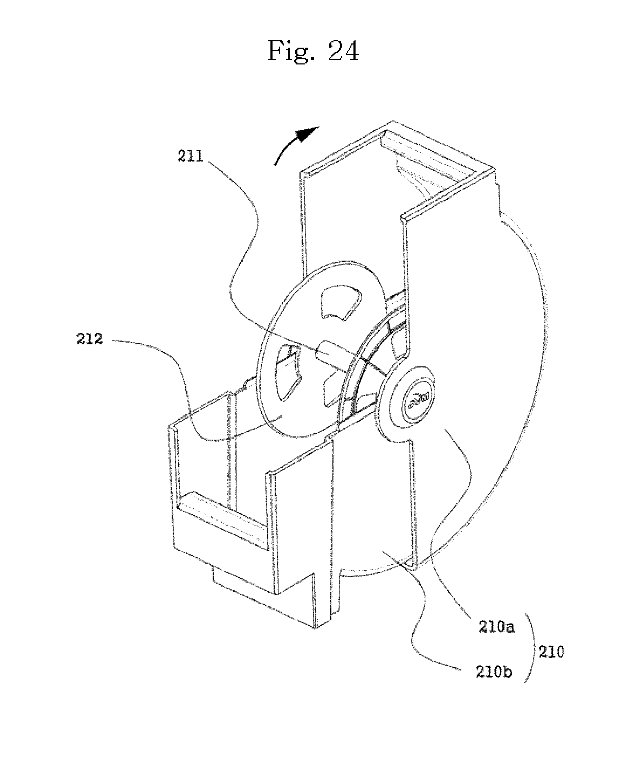

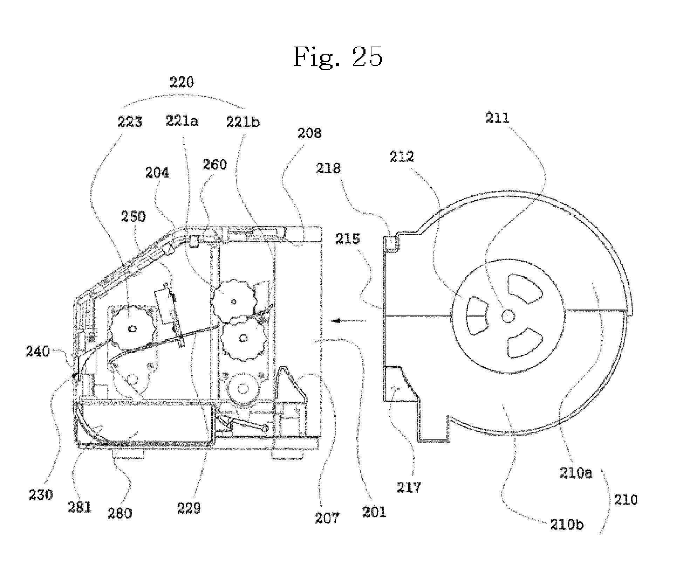

The preservation unit according to another embodiment will be described in detail with reference to FIGS. 24 and 25. FIG. 24 is a perspective view illustrating a state in which the preservation unit is opened according to an embodiment, and FIG. 25 is a cross-sectional view illustrating the state in which the preservation unit is separated from the medicine-taking management apparatus of FIG. 22.

As schematically described above, the preservation unit 210 according to the current embodiment includes a first housing member 210b and a second housing member 210a.

The first housing member 210b may have a semi-cylindrical shape or fan-shaped cylindrical shape that is a portion of a cylindrical shape of the outer appearance of the preservation unit 210. The second housing member 210a may have a shape corresponding to a remaining cylindrical shape except for the first housing member 210b. The first and second housing members 210a and 210b may be mutually rotatably coupled to each other on the same center shaft.

In the preservation unit 210 according to the current embodiment, since the first housing member 210b is disposed under the second housing member 210a, the first and second housing members 210b and 210a may have a cylindrical shape on the whole except for an opening (see reference numeral 215 of FIG. 23).

The second housing member 210a may have an inner diameter equal to or greater than an outer diameter of the first housing member 210b. Thus, the second housing member 210a may be rotated to overlap an outer portion of the first housing member 210b. In this case, an inner space may be exposed to the outside.

A wheel 212 rotatable along a central shaft 211 is disposed within the preservation unit 210. The chartula package may be wound around the central shaft 211 of the wheel 212.

As shown in FIG. 25, each of the preservation unit 210 and the housing 210 has a coupling structure so that the preservation unit 210 and the housing 210 are detachably coupled to each other. In detail, a first protrusion 208 is disposed on an upper portion of the rear surface of the housing 201, and a second protrusion 207 is disposed on a lower portion of the rear surface of the housing 201. The first protrusion 208 may protrude downward from the upper portion of the housing 201, and the second protrusion 207 may protrude upward from the lower portion of the housing 201.

Thus, a first groove 218 and a second groove 217 are defined in a side of the opening 215 of the preservation unit 210. The first groove 218 is recessed downward from an upper portion of the opening 215, and the second groove 217 is recessed upward from a lower portion of the opening 215.

A manager may insert the second protrusion 207 into the second groove 217 and then lift the preservation unit 210 up to couple the first groove 218 to the first protrusion 208.

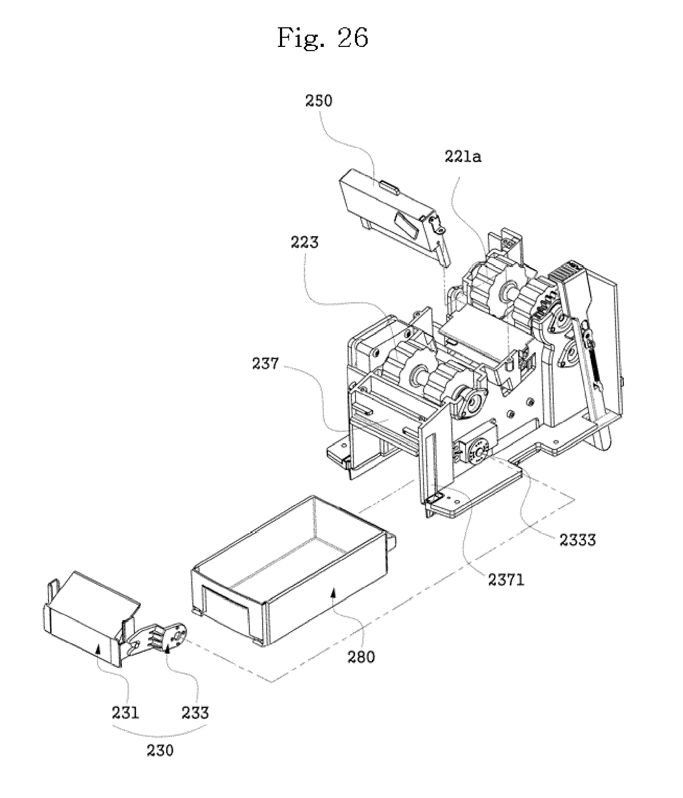

Inner components of the medicine-taking management apparatus according to another embodiment will be described with reference to FIGS. 25 and 26. FIG. 25 is a cross-sectional view illustrating the state in which the preservation unit is separated from the medicine-taking management apparatus of FIG. 22, and FIG. 26 is an exploded perspective view illustrating inner components of the medicine-taking management apparatus of FIG. 22.

The medicine-taking management apparatus according to the current embodiment includes inner components such as a transfer unit, a path selection unit 250, a scan unit 260, and a storage unit 280.

The transfer unit 220 may take the chartula package out of the preservation unit 210 to transfer the chartula package toward a dispensing hole 240. The transfer unit 220 includes a transfer path support plate 229, unwinding rolls 221a and 221b, and a discharge roll 223.

The transfer path support plate 229 has a plate shape. Also, the transfer path support plate 229 is disposed along a transfer path through the chartula package is transferred. The transfer path of the chartula package according to the current embodiment may be gradually inclined downward toward the dispensing hole 240. That is, since the transfer path support plate 229 is inclined downward toward the discharge roll 223 from the unwinding rolls 221a and 221b, the chartula package may descend along the inclination when the chartula package is transferred toward the dispensing hole. Thus, a limitation in which the chartula package or the cut chartula is not transferred by friction or hanging with the support plate 220 or other components may be maximally prevented. Also, since an unnecessary space between the discharge roll 223 and the storage unit 280 is omitted due to the inclined transfer path, the apparatus may be compact in size.

The unwinding rolls 221a and 221b may initially roll the chartula package withdrawn from the preservation unit 210 to transfer the chartula package. That is, when the unwinding rolls 221a and 221b are operated in a state where the foremost end of the chartula package is disposed between the unwinding rolls 221a and 221b, the chartula package may be transferred along a transfer path due to a friction between the unwinding rolls 221a and 221b and the chartula package.

The discharge roll 223 transfers the transferred chartula toward the dispensing hole 240. In detail, the discharge roll 223 may be disposed above the transfer path support plate 229 to roll the chartula or the chartula package when the chartula or the chartula package is disposed between the discharge roll 223 and the transfer path support plate 229, thereby transferring the chartula or the chartula package.

The unwinding rolls 221a and 221b and the discharge roll 223 may be formed of a porous elastic material such as sponge. If each of the unwinding rolls 221a and 221b and the discharge roll 223 does not have elasticity or has less elasticity, medicines accommodated within the chartula package may be damaged. Also, a concave portion and a convex portion of an outer surface of each of the unwinding rolls 221a and 221b and the discharge roll 223 may be alternately disposed. In this case, a contact area between the upper unwinding roll 221a and the lower unwinding roll 221b may increase. Thus, when the chartula package is disposed between the upper and lower unwinding rolls 221a and 221b, the chartula package may be more smoothly transferred due to the increasing friction force.

A motor for driving the upper and lower unwinding rolls 221a and 221b and a motor for driving the discharge roll 223 may be separately provided so that the upper and lower unwinding rolls 221a and 221b and the discharge roll 223 may be independently operated. As described above, when the upper and lower unwinding rolls 221a and 221b and the discharge roll 223 may be independently operated, only a specific roll may be controlled to correct a position of the specific roll when the chartula package is aligned, or an error such as jamming of the chartula package into other components occurs.

As shown in FIG. 25, the cutting unit 250 is disposed in a chartula package transfer path of the transfer unit 220. Particularly, the cutting unit 250 is disposed between the unwinding rolls 221a and 221b and the discharge roll 223 to cut the chartula package transferred along the transfer path. The cutting unit 250 may cut the foremost chartula of the chartula package or cut a plurality of chartulas including the foremost chartula.

The scan part 260 is disposed in the transfer path of the chartula package. The scan part 260 may be disposed between the discharge roll 223 and the unwinding rolls 221a and 221b in the transfer path. The scan part 260 scans a QR code printed on the chartula package and a cutoff position or dotted line of the chartula package. The scan part 260 may be embodied in various manners. That is, a scanner for scanning the two-dimensional graphic code and the dotted line may be separately provided. Alternatively, the two-dimensional graphic code and the dotted line may be photographed and recognized at the same time by using a camera.

A storage unit 280 may be provided as a predetermined space part which is defined under the path selection unit 230 and the transfer unit 220 to store the chartula. As shown in FIG. 25, an inclined guide part may be disposed on a position of the path selection unit 230 at which the chartula drops down. The guide part is not limited to a shape thereof if the guide part is inclined in the dropping direction of the chartula. That is, the guide part 281 may have various shapes with a curved shape or plane shape. As shown in FIG. 26, the path selection unit 230 may be provided in a drawer shape that is withdrawable to the outside.

The medicine-taking management apparatus according to the current embodiment may have two paths through which the chartula is transferred. A first transfer path may be a path through which the chartula withdrawn from the preservation unit 210 is dispensed to the outside through the transfer path support plate 229 and the dispensing hole 240. The second transfer path may be a path through which the chartula withdrawn from the preservation unit 210 drops down from an end of the transfer path support plate 229 and is accommodated in the storage unit 280.

The path selection unit 230 may be operated so that the chartula is transferred along one of the first and second transfer paths. The path selection unit 230 may be connected to a rotation shaft 2333 and constantly rotated. Also, the path selection unit 230 may be vertically guided along a guide groove 2371 of a support part 237 to ascend or descend. Hereinafter, their detailed descriptions will be described.

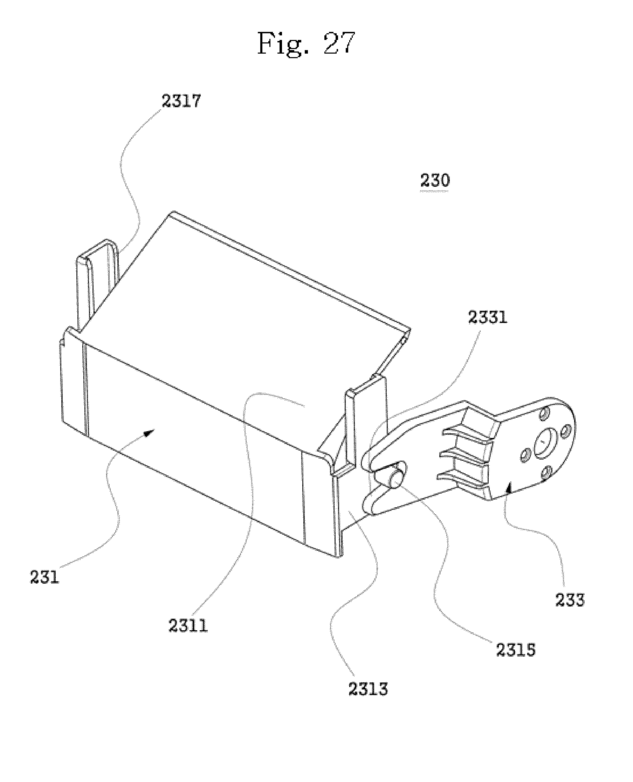

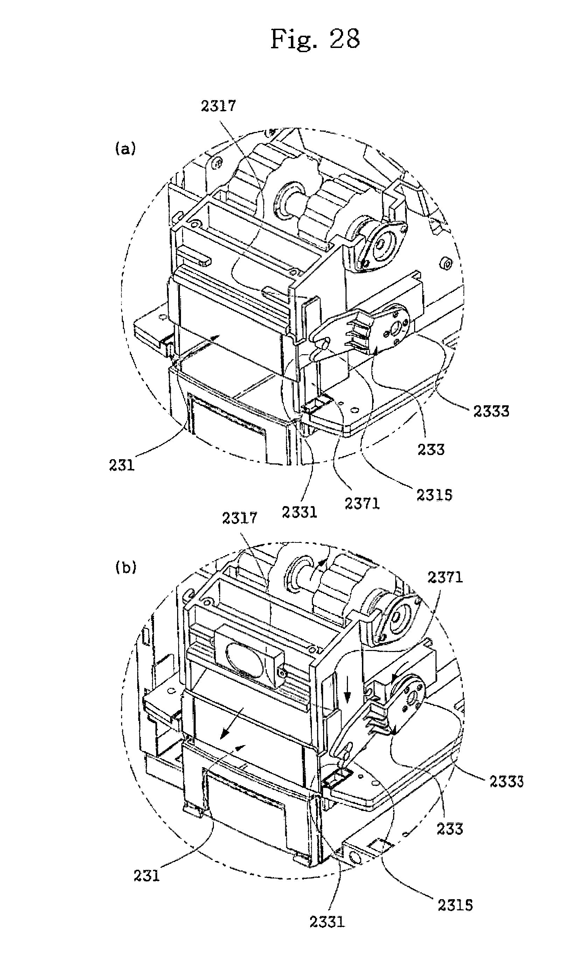

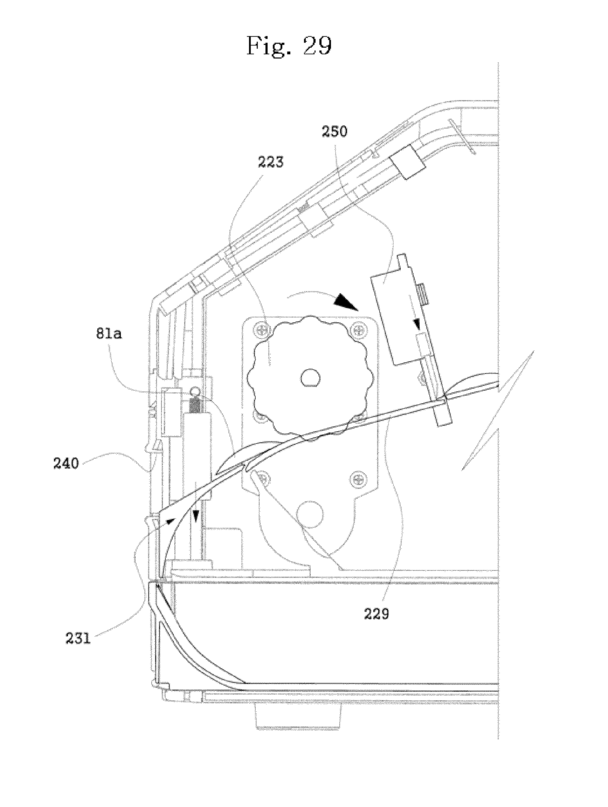

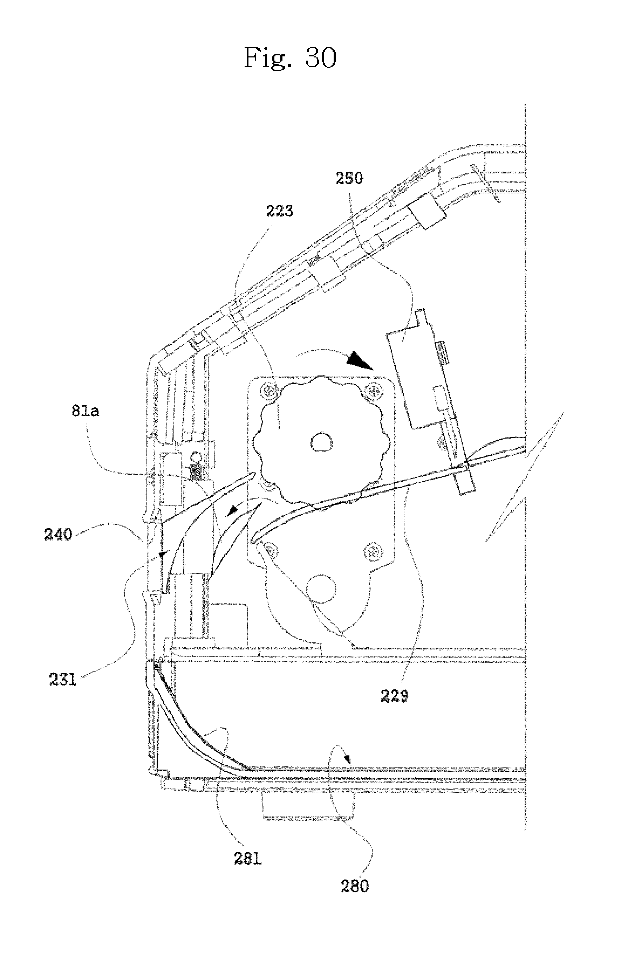

The path selection unit 230 including an extension part and a path selection driving part will be described with reference to FIGS. 27 to 30. FIG. 27 is a perspective view of an extension part and a path selection driving part according to another embodiment, FIG. 28 is a partial perspective view illustrating operations of the extension part and the path selection driving part according to another embodiment, and FIGS. 29 and 30 are cross-sectional view of the extension part according to the operation state in FIG. 28.

The path selection unit 230 includes an extension part 231 and a path selection driving part 233.

Referring to FIG. 27, the extension part 231 includes a body part 2311 and a side part 2313. The body part 2311 has an inclination corresponding to that of the above-described support plate. Also, since the body part 2311 is disposed adjacent to the transfer path support plate, the transfer path may extend or be spaced from the transfer path support plate so that the transfer path is broken off.

The side part 2313 may be disposed on one side surface or both side surfaces of the body part 2311. A guide protrusion 2317 may be disposed inside the side part 2313. The guide protrusion 2317 may vertically protrude with a predetermined length. An outwardly protruding protrusion 2315 to be pressed (hereinafter, referred to as a pressed protrusion) is disposed on an outer surface of the side part 2313.

A rotation arm 233 may be a component that vertically presses the pressed protrusion 2315 to elevate the extension part 231. The rotation arm 233 having a cutoff portion 2331 in which an end of the rotation arm 233 is cut outwardly from a rotation center of the rotation arm 233. The pressed protrusion 2315 is accommodated into the cutoff portion 2331. The pressed protrusion 2315 may receive force for elevating the extension part 231 as the rotation arm 233 is rotated. For convenience of description, the rotation arm 233, the rotation shaft 2333 that serves as the rotation center of the rotation arm 233, and the motor for driving the rotation arm 233 may be commonly called the path selection driving part.

Referring to FIG. 28A, when the rotation arm 233 is rotated in a clockwise direction, the pressed protrusion 2315 accommodated in the cutoff portion 2331 is pressed upward, and thus, the extension part 231 ascends. Here, the guide protrusion 2317 may ascend along the guide groove 2371 to prevent the extension part 231 from being separated.

Referring to FIG. 28B, when the rotation arm 233 is rotated in a counterclockwise direction, the pressed protrusion 2315 accommodated in the cutoff portion 2331 is pressed downward, and thus, the extension part 231 descends. Here, the guide protrusion 2317 may descend along the guide groove 2371 to prevent the extension part 231 from being separated.

Referring to FIG. 29, when the extension part 231 descends, the above-described first transfer path is formed. That is, the chartula 81a cut by the cutting unit 250 may be dispensed along the path defined by the transfer path support plate 229, the extension part 231, and the dispensing hole 240 to the outside by the rolling of the discharge roll 223.

Referring to FIG. 30, when the extension part 231 ascends, the above-described second transfer path is formed. That is, since the extension part 231 ascends, and thus the continuous first transfer path is broken off to form a space through which the chartula drops into the storage unit 280, the second transfer path defined by the transfer path support plate 229 and the storage unit 280 may be formed. When the chartula 81a cut by the cutting unit 250 is rolled by the discharge roll 223, the chartula 81a may not be transferred toward the extension part 231 and the dispensing hole 240, but drop into the storage unit 280. The dropping chartula 81a is seated on the guide part 281 and then introduced into the storage unit 280.

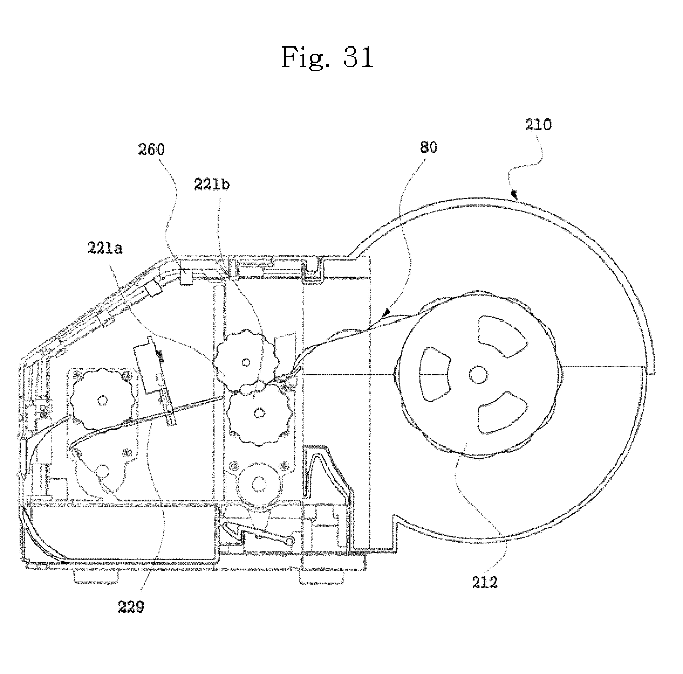

An operation process of the medicine-taking management apparatus according to another embodiment will be described with reference to FIGS. 31 to 36. FIGS. 31 to 36 are cross-sectional views illustrating an operation process of the medicine-taking management apparatus according to another embodiment.

Referring to FIG. 31, a chartula package 80 wound around a wheel 212 within the preservation unit 210 is disposed between the upper unwinding roll 221a and the lower unwinding roll 221b. Then, when the unwinding rolls 221a and 221b are rotated, the chartula package 80 is dispensed.

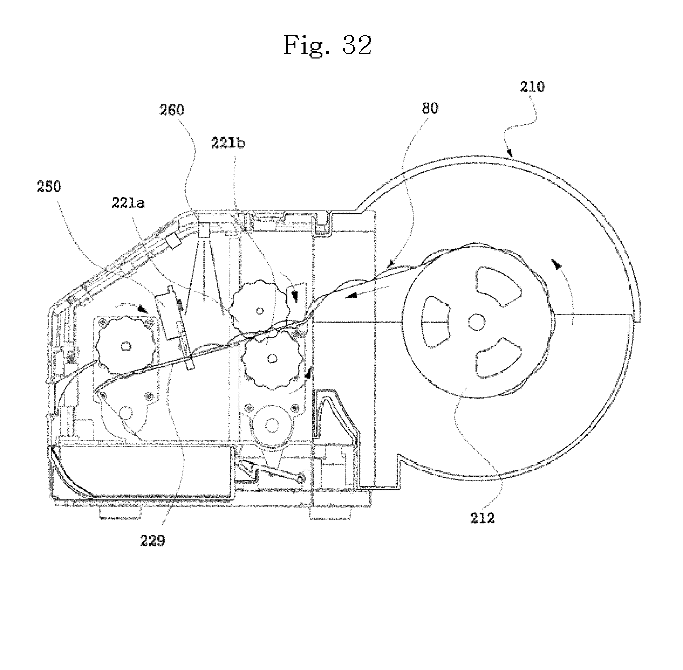

As shown in FIG. 32, the chartula package 80 is transferred toward the cutting unit 250 along the transfer path support plate 229. Here, the scan part 260 scans a two-dimensional graphic code printed on the chartulas included in the chartula package 80.

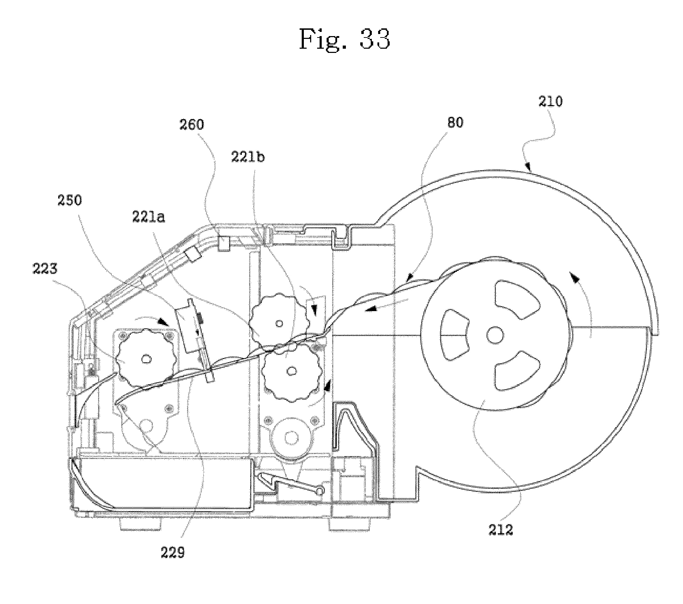

When the chartula package 80 is further transferred, as shown in FIG. 33, the chartula package 80 is transferred up to the discharge roll 223 via the cutting unit 250. Here, the cutting unit 250 may cut a dotted line between the chartulas by using the information scanned by the scan part 260. In this case, the chartulas continuously connected to each other are unwound from the wheel 212.

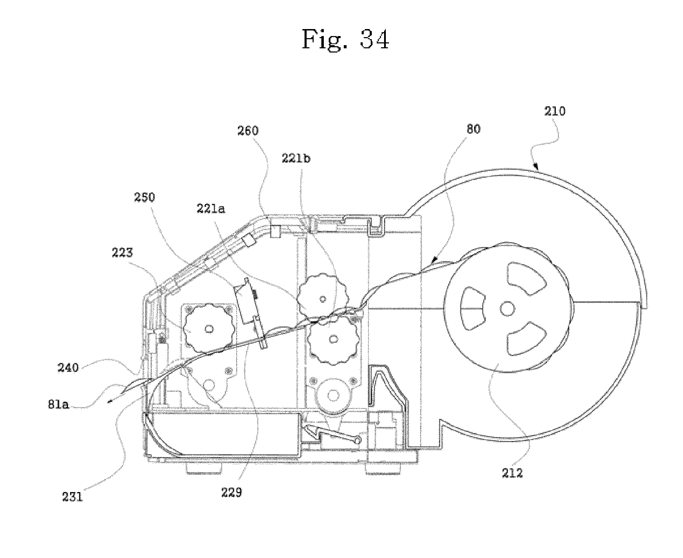

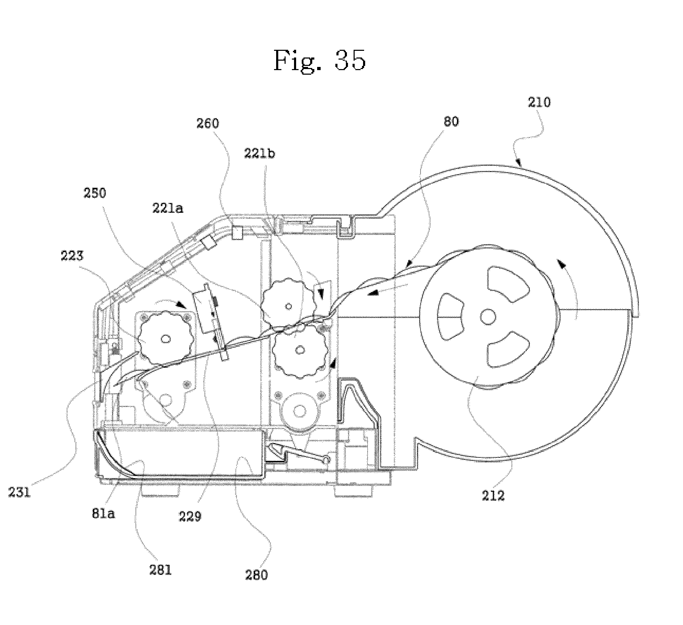

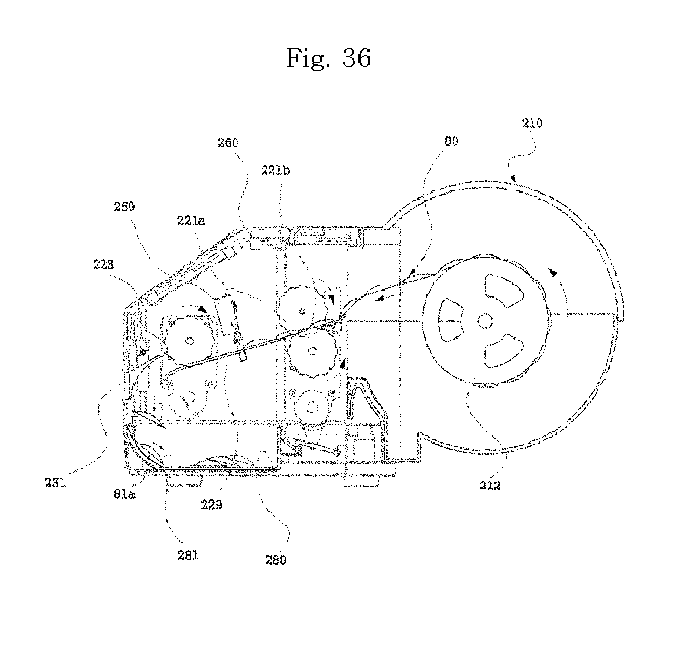

As described above, the foremost chartula 81a cut as the extension part 231 is operated may travels along the two paths. That is, as shown in FIG. 34, when the extension part 231 and the transfer path support plate 229 form the continuous path, the chartula 81a is dispensed to the outside through the dispensing hole 240 via an inclined plane of the extension part 231. On the other hand, as shown in FIG. 35, when the extension part 231 is separated from the continuous path connected to the transfer path support plate 229, the chartula 81a may drop through a lower gap of the extension part 231. As shown in FIG. 36, the dropping chartula 81a is seated on the inclined guide part 81a and then accommodated into the storage unit 280.

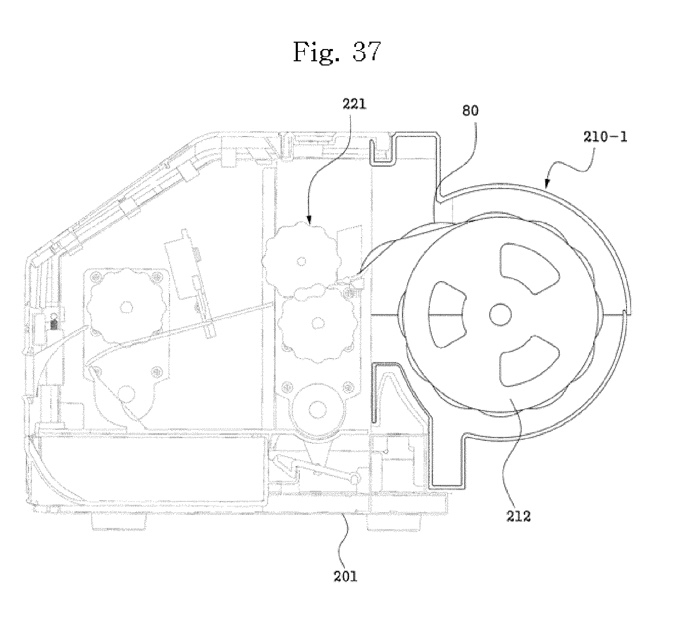

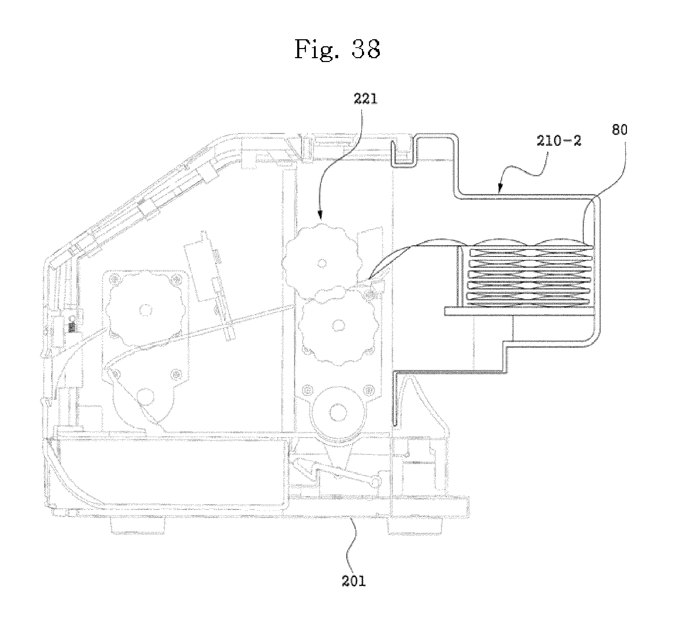

Referring to FIGS. 37 and 38, the preservation unit according to the present invention may be verified in shape and size. For example, when the preservation unit 210 according to the foregoing embodiment has a size enough to accommodate the chartula package, as shown in FIG. 37, a preservation unit 210-1 for packing chartula packages for 15 days may have a relative small size. This embodiment is the same as the foregoing embodiment in that a chartula package 80 wound around a wheel within the preservation unit 210-1 is transferred by a unwinding roll 221.

Alternatively, in a case of a chartula package containing the too small number of chartulas to be stored in a wound state, such as chartula packages for days less than those of the preservation unit 210-1 of FIG. 39, for example, chartula package for about 7 days, the chartula packages may be stored in a simple stacked state as shown in FIG. 38. That is, a preservation unit 210-2 may have a box shape with the same size as that enough to accommodate the chartula packages for 7 days.

As described above, the preservation unit for packing or accommodating the chartula package may vary in shape or size according to the number of days of prescription at pharmacy or a place at which the chartula package is prepared according to the prescription. The user may simply mount the various preservation units on the housing 201 to utilize the various preservation units.

According to the present invention, the patient's medicine-taking may be managed so that the patient takes a medicine at an adequate medicine-taking time.

Also, according to the present invention, even though the medicine is not taken at a fixed medicine-taking time when the medicine-taking time is fixed, the follow-up medicine-taking management may be enabled without separately managing the corresponding chartula by the user.

Also, according to the present invention, since the various information that is capable of determining whether the patient takes the medicine in addition to the medicine-taking history information, the follow-up management for the patient may be accurately performed.

Also, according to the present invention, since the preservation unit for storing the chartula package is detachably provided on the medicine-taking management apparatus, the medicine may be easily supplemented or replaced.

Also, according to the present invention, since the transfer path is inclined downward toward the dispensing direction, it may prevent the chartula package or a portion of the cut chartula package from being hung to easily dispense the chartula package. In addition, the medicine-taking management apparatus may be miniaturized.

Although exemplary embodiments of the present invention are described, the technical spirit of the present invention is not limited to the above-described exemplary embodiments, and thus various dispensing boxes for the drug-containing ampoule and the dispensing apparatus including the same can be realized without departing form the spirit or scope of the invention.

## Claims

1. A medicine-taking management apparatus comprising:
a preservation unit for preserving a chartula package;
a transfer unit comprising at least one roll for transferring the preserved chartula package;
a cutting unit for cutting a chartula of the transferred chartula package;
a dispensing hole through which the cut chartula is dispensed from the chartula package to the outside;
a storage unit providing a space in which the cut chartula is stored therein; and
a path selection unit for controlling a path so that the cut chartula travels to one of the dispensing hole and the storage unit.

2. The medicine-taking management apparatus of claim 1, further comprising a housing comprising a detachment part to which the preservation unit is detached.

3. The medicine-taking management apparatus of claim 2, wherein the preservation unit is provided in plurality with various sizes and shapes according to the number of days of prescription, and the plurality of preservation units are selectively mounted on the housing.

4. The medicine-taking management apparatus of claim 1, wherein the path selection unit comprises:
a support plate providing a path so that the cut chartula is transferred up to the dispensing hole;
a rotation shaft disposed parallel to a transfer direction of the cut chartula; and
a connection arm connecting the rotation shaft to the support plate, the connection arm being rotated so that the support plate is inclined at a predetermined angle or more.

5. The medicine-taking management apparatus of claim 1, further comprising a transfer path support plate providing a path so that the cut chartula is transferred up to the dispensing hole;
wherein the path selection unit comprises:
an extension part disposed between the transfer path support plate and the dispensing hole so that the transfer path extends; and
a path selection driving part moving the extension part between the transfer path support plate and the dispensing hole to form a first transfer path of the cut chartula, which are defined by the transfer path support plate, the extension part, and the dispensing hole and separating the extension part from a space between the transfer path support plate and the dispensing hole to form a second transfer path for allowing the cut chartula to drop into the space between the transfer path support plate and the dispensing hole.

6. The medicine-taking management apparatus of claim 5, further comprising a support part having an elevation guide groove having a groove shape with a predetermined length in a vertical direction,
wherein the extension part comprises a body part continuously forming the transfer path together with the support plate, a side part extending from each of both side surfaces of the body part, a guide protrusion protruding inward from the side part and guided in a state where the guide protrusion is inserted into the elevation guide groove, and a protrusion to be pressed, which protrudes outward from the side part,
the path selection driving part comprises a pair of rotation arms that are rotated about a rotation shaft disposed perpendicular to the transfer path and the elevation direction of the extension part, and
each of the rotation arms has a cutoff portion, of which a portion is cut to accommodate the protrusion to be pressed, on an outer end thereof.

7. The medicine-taking management apparatus of claim 1, wherein the transfer unit comprises:
a discharge roll being rolled to discharge the cut chartula; and
an unwinding roll for transferring the chartula package toward the dispensing hole, and
at least one outer surface of the discharge roll and the unwinding roll has an outer surface formed of a porous elastic material.

8. The medicine-taking management apparatus of claim 7, wherein at least one the discharge roll and the unwinding roll has a concave portion and a convex portion which are alternately disposed along an outer surface thereof.

9. The medicine-taking management apparatus of claim 1, wherein the storage unit comprises an inclined guide part so that the cut chartula dropping from the path selection unit is seated.

10. The medicine-taking management apparatus of claim 1, further comprising a scan part for scanning a cutting position of the chartula package transferred by the transfer unit and a two-dimensional graphic code printed on the chartula package.

11. The medicine-taking management apparatus of claim 10, further comprising a control part for recognizing at least one medicine-taking information of patient identification data, a medicine-taking time, and chartula package information from the two-dimensional graphic code scanned by the scan part.

12. The medicine-taking management apparatus of claim 11, wherein the control part controls the transfer unit, the cutting unit, and the path selection unit to store a corresponding chartula into the storage unit when a specific chartula corresponding to a specific medicine-taking time is not dispensed through the dispensing hole for the specific medicine-taking time.

13. The medicine-taking management apparatus of claim 11, further comprising an imaging part for photographing an external image; and
a communication part,
wherein the control part compares the medicine-taking information comprising the medicine-taking time and the chartula package information that are recognized from the two-dimensional graphic code scanned by the scan part to an actually dispensed time of the chartula through the dispensing hole and information of the actually dispensed chartula, and
a communication part transmits the result obtained by the comparing the medicine-taking information to the medicine-taking history information through the control part to at least one of a guardian terminal and a management server.

14. The medicine-taking management apparatus of claim 1, wherein the transfer unit is disposed so that the transfer path is inclined downward toward the dispensing hole.

15. The medicine-taking management apparatus of claim 1, wherein the storage unit is provided in a drawer type that is withdrawable to the outside.
